(19) Europäisches Patentamt
European Patent Office
Office européen des brevets

(11) Veröffentlichungsnummer: **0 658 559 A1**

# (12) EUROPÄISCHE PATENTANMELDUNG

(21) Anmeldenummer: **94119114.0**

(22) Anmeldetag: **05.12.94**

(51) Int. Cl.$^6$: **C07D 513/04**, A61K 31/54, //(C07D513/04,333:00,279:00)

(30) Priorität: **14.12.93 AT 2530/93**
**14.12.93 AT 2531/93**
**18.05.94 AT 1026/94**

(43) Veröffentlichungstag der Anmeldung:
**21.06.95 Patentblatt 95/25**

(84) Benannte Vertragsstaaten:
**AT BE CH DE DK ES FR GB GR IE IT LI LU NL PT SE**

(71) Anmelder: **CHEMISCH PHARMAZEUTISCHE FORSCHUNGS-GESELLSCHAFT m.b.H.**
**St. Peter-Strasse 25**
**A-4020 Linz (AT)**

(72) Erfinder: **Binder, Dieter, Prof. Dr.**
**Sieveringerstrasse 207**
**A-1190 Wien (AT)**
Erfinder: **Weinberger, Josef, Dipl.-Ing. Dr.**
**Schüttelstrasse 15a/19**
**A-1020 Wien (AT)**

(54) **Thienothiazinderivate, Verfahren zu ihrer Herstellung und ihre Verwendung als 5-dipoxygenase und Cyclooxygenaseinhibitoren.**

(57) Neue Thienothiazin-Derivate der allgemeinen Formel

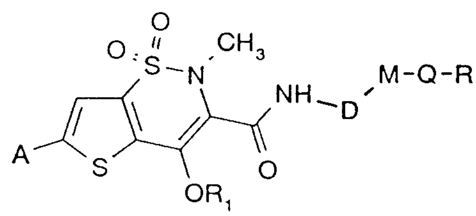

(I),

worin:
**A** Niederalkyl, perfluoriertes Niederalkyl, Niederalkyloxy, Halogen, Nitro, Cyano oder einen mono- oder polycyclischen 5-12 gliedrigen, gegebenenfalls teilweise hydrierten Aryl- oder Heteroarylrest mit 1-4 Heteroatomen wie O, S und N, der gegebenenfalls mit Niederalkyl, Perfluorniederalkyl, Aryl, Heteroaryl, substituiertem Aryl, substituiertem Heteroaryl, Halogen, Niederalkoxy, Aryloxy, substituiertem Aryloxy, Heteroaryloxy, substituiertem Heteroaryloxy und Nitro substituiert sein kann, in denen die angesprochenen Substituenten in den substituiertem Aryl, substituiertem Heteroaryl, substituiertem Aryloxy und substituiertem Heteroaryloxy Halogen, Niederalkyl, Perfluorniederalkyl, Niederalkoxy u.ä. bedeuten;
**D** einen 2-Pyridylrest oder einen Rest

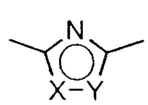

,

wobei **X** und **Y** unabhängig voneinander CH, $NR^6$, O oder S mit $R^6$ Wasserstoff oder Niederalkyl bedeuten,
**M** eine Einfachbindung, eine geradkettige oder verzweigte Kohlenstoffkette mit 1-12 Kohlenstoffatomen in der Kette, wobei diese Kette ein oder mehrere Doppel- und/oder Dreifachbindungen und/oder ein oder mehrere Heteroatome, wie O, S und N, enthalten kann, einen 5-12 gliedrigen mono- oder polycyclischen gegebenenfalls

EP 0 658 559 A1

teilweise hydrierten Aryl- oder Heteroarylrest mit 1-4 Heteroatomen wie O, S und N, der durch Halogen, Niederalkyl oder Niederalkoxy substituiert sein kann;
**Q** eine Einfachbindung oder ein Heteroatom wie O, S und N;
**R** Wasserstoff, einen 5-12 gliedrigen mono- oder polycyclischen Aryl- oder Heteroarylrest mit 1-4 Heteroatomen wie O, S und N, der gegebenenfalls teilweise hydriert sein kann, oder auch ein oder mehrfach mit Halogen, Niederalkyl oder Niederalkoxy substituiert sein kann;
und **$R_1$** Wasserstoff, oder

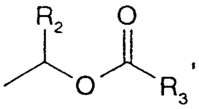
,

worin **$R_2$** Niederalkyl und **$R_3$** Niederalkyl, Aryl
oder $-OR_4$ mit **$R_4$** Niederalkyl, Cycloalkyl mit 4-8 Kohlenstoffatomen oder Aryl;
bedeuten, sowie ihre pharmazeutisch verwendbaren Salze.

Die vorliegende Erfindung betrifft neue, therapeutisch wertvolle Thienothiazin-Derivate der allgemeinen Formel

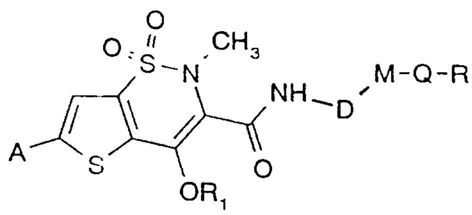

(I),

worin:

**A** Niederalkyl, perfluoriertes Niederalkyl, Niederalkyloxy, Halogen, Nitro, Cyano oder einen mono- oder polycyclischen 5-12 gliedrigen, gegebenenfalls teilweise hydrierten Aryl- oder Heteroarylrest mit 1-4 Heteroatomen wie O, S und N, der gegebenenfalls mit Niederalkyl, Perfluorniederalkyl, Aryl, Heteroaryl, substituiertem Aryl, substituiertem Heteroaryl, Halogen, Niederalkoxy, Aryloxy, substituiertem Aryloxy, Heteroaryloxy, substituiertem Heteroaryloxy und Nitro substituiert sein kann, in denen die angesprochenen Substituenten in den substituiertem Aryl, substituiertem Heteroaryl, substituiertem Aryloxy und substituiertem Heteroaryloxy Halogen, Niederalkyl, Perfluorniederalkyl, Niederalkoxy u.ä. bedeuten;

**D** einen 2-Pyridylrest oder einen Rest

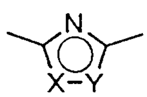

,

wobei **X** und **Y** unabhängig voneinander CH, $NR^6$, O oder S mit $R^6$ Wasserstoff oder Niederalkyl bedeuten,

**M** eine Einfachbindung, eine geradkettige oder verzweigte Kohlenstoffkette mit 1-12 Kohlenstoffatomen in der Kette, wobei diese Kette ein oder mehrere Doppel- und/oder Dreifachbindungen und/oder ein oder mehrere Heteroatome, wie O, S und N, enthalten kann, einen 5-12 gliedrigen mono- oder polycyclischen gegebenenfalls teilweise hydrierten Aryl- oder Heteroarylrest mit 1-4 Heteroatomen wie O, S und N, der durch Halogen, Niederalkyl oder Niederalkoxy substituiert sein kann;

**Q** eine Einfachbindung oder ein Heteroatom wie O, S und N;

**R** Wasserstoff, einen 5-12 gliedrigen mono- oder polycyclischen Aryl- oder Heteroarylrest mit 1-4 Heteroatomen wie O, S und N, der gegebenenfalls teilweise hydriert sein kann, oder auch ein oder mehrfach mit Halogen, Niederalkyl oder Niederalkoxy substituiert sein kann;

und $\mathbf{R_1}$ Wasserstoff, oder

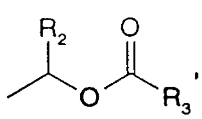

,

worin $\mathbf{R_2}$ Niederalkyl und $\mathbf{R_3}$ Niederalkyl, Aryl

oder $-OR_4$ mit $\mathbf{R_4}$ Niederalkyl, Cycloalkyl mit 4-8 Kohlenstoffatomen oder Aryl; bedeuten, sowie ihre pharmazeutisch verwendbaren Salze.

Ein weiterer Gegenstand der vorliegenden Erfindung ist ein Verfahren zur Herstellung von Verbindungen der allgemeinen Formel (I), welches dadurch gekennzeichnet ist, das man eine Verbindung der allgemeinen Formel

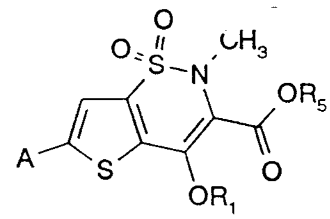

(II),

worin $R_1$ Wasserstoff, $R_5$ Niederalkyl bedeutet und **A** obige Bedeutung hat, mit einer Verbindung der allgemeinen Formel

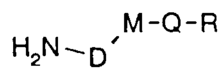

(III),

worin **D**, **M**, **Q** und **R** obige Bedeutung haben, umsetzt und die so erhaltenen Verbindungen der allgemeinen Formel (I) für $R_1$ = Wasserstoff gegebenenfalls in ihre pharmazeutisch verwendbaren Salze überführt oder mit einer Verbindung der allgemeinen Formel

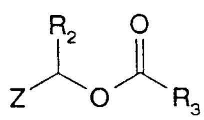

(IV),

worin **Z** Chlor oder Brom bedeutet und $R_2$ und $R_3$ obige Bedeutung haben, zu den Verbindungen der allgemeinen Formel (I) mit $R_1$ nicht Wasserstoff umsetzt.

Der oben verwendete Ausdruck "Aryl" kann beispielsweise Phenyl, Naphthyl, u.ä. und der Ausdruck "Heteroaryl" kann bespielsweise Thienyl, Furyl, Pyrrolyl, Pyridinyl, Pyrimidinyl, Pyridazinyl, Imidazolyl, Pyrazolyl, Thiazolyl, Oxazolyl, Oxadiazolyl, Thiadiazolyl, Pyranyl, Thiopyranyl, Benzo[b]furyl, Benzo[b]-thienyl, Chinolinyl, iso-Chinolinyl und dergleichen bedeuten.

Der oben verwendete Ausdruck "Halogen" bedeutet Fluor, Chlor, Brom oder Iod.

Der oben verwendete Ausdruck "Niederalkyl" bedeutet einen geradkettigen oder verzweigten Alkylrest mit 1-4 Kohlenstoffatomen, beispielsweise Methyl, Ethyl, n- und i-Propyl, n-, i- und t-Butyl.

Der oben verwendete Ausdruck "Niederalkyloxy" bedeutet einen geradkettigen oder verzweigten Alkoxyrest mit 1-4 Kohlenstoffatomen, beispielsweise Methoxy, Ethoxy, n- und i-Propoxy, n-, i- und t-Butoxy.

Die erfindungsgemäßen Umsetzungen werden am besten so durchgeführt, daß man entweder

A) die Verbindung der allgemeinen Formel (II) in einem inerten Lösungsmittel, wie Diethylether, THF, Dioxan, Toluol, Benzol usw., löst und bei einer Temperatur zwischen -20 °C und 100 °C ein Äquivalent einer starken Base, wie Butyllithium oder LDA, zusetzt, zu dieser Salzlösung 1-10 Äquivalente einer Verbindung der allgemeinen Formel (III) zusetzt, mindestens ein weiteres Äquivalent der starken Base zusetzt und zwischen 0,5 und 60 Stunden bei -20 °C bis 100 °C rührt, oder

B) eine Lösung der Verbindungen (II) und (III) in einem inerten, hochsiedenden Lösungsmittel, wie Toluol, Xylol, Pyridin, Chinolin, DMF, DMSO oder HMPA usw., 1-30 Stunden zwischen 100 °C und 200 °C erhitzt, die so erhaltenen Verbindungen der allgemeinen Formel (I) werden für den Fall $R_1$ nicht Wasserstoff entweder

C) in einem inerten Lösungsmittel, wie Aceton, DMF, DMSO oder HMPA mit mindestens einem Äquivalent einer Base, wie NaH, Natriumtrimethylsilanolat u.ä., bei Raumtemperatur umgesetzt und 1-100 Stunden bei 0-150 °C mit mindestens einem Äquivalent einer Verbindung der allgemeinen Formel (IV) gerührt oder

D) in einem basischen Lösungsmittel wie Triethylamin, Pyridin, Chinolin u.ä., mit mindestens einem Äquivalent einer Verbindung der allgemeinen Formel (IV) 1-100 Stunden bei 0-150 °C gerührt.

Die bei dieser Umsetzung erhaltenen Verbindungen der allgemeinen Formel (I) mit $R_1$ = Wasserstoff sind saure Verbindungen und können auf übliche Weise mit anorganischen oder organischen Basen in ihre pharmazeutisch verträglichen Salze überführt werden.

Die Salzbildung kann beispielsweise durchgeführt werden, indem man die Verbindungen der Formel (I) in einem geeigneten Lösungsmittel, z.B. Wasser, einem niederen aliphatischen Alkohol, THF, Dioxan, Benzol, Diethylether, DMF oder DMSO löst, eine äquivalente Menge der gewünschten Base Zusetzt, für eine gute Durchmischung sorgt und nach beendeter Salzbildung das Lösungsmittel im Vakuum abzieht. Gegebenenfalls können die Salze nach der Isolierung umkristallisiert werden.

Pharmazeutisch verwendbare Salze sind z.B. Metallsalze, insbesondere Alkalimetall- und Erdalkalimetallsalze, wie Natrium-, Kalium-, Magnesium-oder Calciumsalze. Andere pharmazeutische Salze sind beispielsweise leicht kristallisierende Ammoniumsalze. Letztere leiten sich ab von Ammoniak oder von organischen Aminen, z.B. Mono-, Di- oder Tri-nieder-(alkyl, cycloalkyl oder hydroryalkyl)-aminen, Niederalkylendiaminen oder (Hydroxy-niederalkyl oder Aryl-niederalkyl)-niederalkylammoniumbasen, z.B. Methylamin, Diethylamin, Triethylamin, Dicyclohexylamin, Triethanolamin, Ethylendiamin, Tris(hydroxymethyl)-aminomethan, Benzyltrimethylammoniumhydroxid und dergleichen ab. Die Verbindungen der allgemeinen Formel (II), (III) und (IV) sind literaturbekannt oder können analog dazu nach üblichen und dem Fachmann geläufigen Methoden hergestellt werden.

Die erfindungsgemäßen Verbindungen der allgemeinen Formel (I) und deren Salze sind oral wirksam und zeigen im Vergleich zu 6-Chlor-2-methyl-N-(2-pyridyl)-2H-thieno[2,3-e]-1,2-thiazin-3-carbonsäureamid-1,1-dioxid ("*LORNOXICAM*", US-Patent 4,180,662) überraschender Weise eine signifikant erhöhte Hemmung der 5-Lipoxygenase bei weitgehendster Beibehaltung der Cyclooxygenasehemmung.
Sie sind daher zur Behandlung von Beschwerden die durch das natürliche Produkt der 5-Lipoxygenase nämlich dem Leucotrien-$B_4$ und den Cyclooxygenaseprodukten mitverursacht werden, wie z.B. Entzündung und Schmerz bei allergischem Asthma, Arthritis, Hautallergie, rheumatische Beschwerden usw., besonders gut geeignet.

Aufgrund dieser pharmakologischen Eigenschaften können die neuen Verbindungen allein oder in Mischung mit anderen Wirksubstanzen in Form üblicher galenischer Zubereitungen als Heilmittel zur Behandlung von Erkrankungen, die durch Hemmung der 5-Lipoxygenase und der Cyclooxygenase geheilt oder gelindert werden, Verwendung finden.

Die Erfindung bezieht sich weiterhin auf Heilmittel, die z.B. in Form pharmazeutischer Präparate Verwendung finden, welche die erfindungsgemäßen Verbindungen der allgemeinen Formel (I) und ihre Salze in Mischung mit einem für die orale, enterale, parenterale und topicale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten.

Die pharmazeutischen Präparate können in fester Form z.B. als Tabletten, Filmtabletten, Dragees, Suppositorien, Kapseln, Mikrokapseln oder in flüssiger Form z.B. als Lösungen, Injektionslösungen, Suspensionen oder Emulsionen oder in Zusammensetzungen mit verzögerter Freigabe des Wirkstoffes vorliegen. Gegebenfalls sind sie sterilisiert und/oder enthalten Hilfsstoffe, wie Konservierungs-, Stabilisierungs- oder Emulgiermittel, Salze zur Veränderung des osmotischen Druckes oder Puffer.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.

Die neuen Verbindungen können in den erfindungsgemäßen pharmazeutischen Zusammensetzungen in einem Anteil von etwa 4-200 mg pro Tablette vorhanden sein, wobei der Rest ein pharmazeutisch annehmbarer Füllstoff ist.

Eine geeignete Dosis zur Verabreichung der neuen Verbindungen beträgt etwa 1-200 mg/kg pro Tag, jedoch kommen, je nach dem Zustand des zu behandelnden Patienten, auch andere Dosen in Frage. Die neuen Verbindungen können in mehreren Dosen und auf oralem Weg verabreicht werden.

Die folgenden Beispiele erläutern die Erfindung näher, ohne das diese darauf beschränkt sein soll:

**Beispiel 1**

**6-Chlor-4-hydroxy-2-methyl-N-(2-thiazolyl)-2H-thieno[2,3-e]-1,2-thiazin-3-carboxamid-1,1-dioxid**

1.32g (4.25 mMol) 6-Chlor-4-hydroxy-2-methyl-2H-thieno[2,3-e]-1,2-thiazin-3-carbonsäuremethylester-1,1-dioxid und 0.47g (4.30 mMol) 2-Thiazolamin werden in 12 ml Xylol abs. 17.5 Std. zum Sieden erhitzt. Nach Abziehen des Lösungsmittels wird das Rohprodukt mit 5x20 ml eiskaltem Diethylether digeriert, in 1N NaOH-Lösung aufgenommen, mit Aktivkohle heiß filtriert, angesäuert und der Niederschlag abfiltriert und getrocknet. Ausbeute: 0.175g gelbe Kristalle (10.94 % d.Th.)

Fp: 223-225°C ($H_2O$)
DC: Bz/Dx/MeOH 10:1:1 Rf:0.68
$^1$H-NMR: (DMSO-$d_6$)
δ (ppm) = 7.66 (s; 1H, Thio-H7); 7,60 (d, 1H, Thaz-H5); 7.22 (d; 1H, Thaz-H4); 2.96 (s; 3H, N-$CH_3$)
$^{13}$C-NMR: (DMSO-$d_6$)
δ (ppm) = 166.09; 164.78; 156.45; 138.41; 136.91; 135.23; 127.29; 123.00; 112.95; 111.92; 38.82

**Beispiel 2**

**6-Chlor-4-hydroxy-2-methyl-N-(4-phenyl-2-thiazolyl)-2H-thieno-[2,3-e]-1,2-thiazin-3-carboxamid-1,1-dioxid**

1.37g (4.417 mMol) 6-Chlor-4-hydroxy-2-methyl-2H-thieno[2,3-e]-1,2-thiazin-3-carbonsäure-methylester-1,1-dioxid und 0.78g (4.426 mMol) 4-Phenyl-2-thiazolamin werden in 12.6 ml Xylol abs. zum Sieden erhitzt. Nach 3.5 Std. und 5.5 Std. werden jeweils 0.4g (2.269 mMol) 4-Phenyl-2-thiazolamin zugesetzt.
Nach 6 Std wird das LM abgezogen. Das Rohprodukt wird mit 3x20ml eiskaltem Diethylether digeriert, mit heißem Ethylalkohol gewaschen und aus Methylenchlorid/Aktivkohle umkristal-lisiert.
Ausbeute: 1.15g gelbe Kristalle (57.5 % d.Th.)
Fp: 241-244°C ($CH_2Cl_2$)
DC: $CH_2Cl_2$/MeOH 40:1 Rf: 0.30
$^1$H-NMR: (DMSO-$d_6$)
δ (ppm) = 7.88 (d; 2H, Ph-H2,6); 7.68 (s; 1H, Thio-H7); 7.60 (s; 1H, Thiaz-H5); 7,54-7,33 (m; 3H, Ph-H3,4,5); 2.94 (s; 3H, N-$CH_3$)
$^{13}$C-NMR: (DMSO-$d_6$)
δ (ppm) = 165.58; 161.93; 155.16; 142.72; 136.93; 136.39; 135.50; 131.11; 128.84; 128.66; 125.77; 122.77; 111.11; 108.08; 38.78

**Beispiel 3**

**6-(2-Furyl)-4-hydroxy-2-methyl-N-(4-phenyl-2-thiazolyl)-2H-thieno-[2,3-e]-1,2-thiazin-3-carboxamid-1,1-dioxid**

0.49g (1.459 mMol) 6-(2-Furyl)-4-hydroxy-2-methyl-2H-thieno-[2,3-e]-1,2-thiazin-3-carbonsäure-methylester-1,1-dioxid und 0.77g (4.386 mMol) 4-Phenyl-2-thiazolamin werden in 5 ml Xylol abs. 6 Std. zum Sieden erhitzt. Nach Abziehen des Lösungs-mittels wird das Rohprodukt mit 3x10 ml eiskaltem Ethanol digeriert und aus Dichlormethan/Aktivkohle umkristallisiert.
Ausbeute: 0.34g gelbe Kristalle (48.0 % d.Th.)
Fp: ab 240°C Zers. ($CH_2Cl_2$)
DC:
$CH_2Cl_2$/MeOH 50:1 Rf: 0.23
Bz/Dx/MeOH 10:1:1 Rf: 0.71
$^1$H-NMR: (DMSO-$d_6$)
δ (ppm) = 7,93-7.81 (m, 4H, Thio-H7, Ph-H2,6, Fu-H5); 7,64 (s, 1H, Thaz-H5); 7,53-7,31 (m, 3H, Ph-H3,4,5); 7,22 (d, 1H, Fu-H3); 6,71 (dd, 1H, Fu-H4); 2.98 (s; 3H, N-$CH_3$)
$^{13}$C-NMR: (DMSO-$d_6$)
δ (ppm) =165.71; 161.26; 155.92; 146.93; 144.46; 143.85; 138.62; 138.52; 134.60; 131.82; 128.84; 128.48; 125.77; 117.53; 112.90; 110.77; 109.42; 108.10; 38.93

Das Ausgangsmaterial kann wie folgt hergestellt werden:

**4-Methylbenzolsulfonsäure, [2-cyano-1-(2-furyl)ethenyl]-ester**

Zu 45.75 g (339 mMol) b-Oxo-2-furanpropannitril und 47.95 g (474 mMol) N-Methylmorpholin in 100 ml absolutem Dichlormethan werden bei -5 bis 0°C 67.78 g (356 mMol) p-Toluolsulfonsäurechlorid in 100 ml absolutem Dichlormethan getropft und eine Stunde nachgerührt. Das Lösungsmittel wird abgezogen, der Rückstand zwischen 500 ml Essigsäureethylester und 400 ml 1n Salzsäure verteilt und die wäßrige Phase mit 6x150 ml Essigsäureethylester extrahiert. Die vereinten Extrakte werden über $Na_2SO_4$/Aktivkohle getrocknet, filtriert und das Extraktionsmittel abdestilliert. Den erhaltenen Feststoff kristallisiert man aus 150 ml Toluol/Aktivkohle um.

Ausbeute: 92.48 g farblose Kristalle (94% d.Th.)
DC: *LM*...Bz:$Et_2O$ = 10:1; 0.65
Fp.: 109-111 °C(Toluol)
$^1$H-NMR: (DMSO-$d_6$)

d(ppm): 8.00-7.85 (m, 3H, Bz-H2,6, Fu-H5); 7.60-7.48 (m, 2H, Bz-H3,5) 6.74 (dd, 1H, Fu-H3*); 6.57 (dd, 1H, Fu-H4*); 6.33 (s, 1H, CH-CN); 2.45 (s, 3H, $CH_3$)

$^{13}$C-NMR: (DMSO-$d_6$) d(ppm): 150.2; 147.7; 146.8; 145.4; 131.1; 130.4; 128.4; 115.9; 114.4; 113.1; 87.3; 21.2

**3-Amino-5-(2-furyl)-2-thiophencarbonsäure.methylester Hydrochlorid**

16.75 g (309 mMol) Natriummethanolat in 750 ml absolutem Methanol werden bei 15-20 °C mit 32.75 g (309 mMol) Thioglycolsäuremethylester versetzt und 20 Minuten bei Raumtemperatur gerührt. Anschließend gibt man in einer Portion 74.38 g (257 mMol) 4-Methylbenzolsulfonsäure-[2-cyano-1-(2-furyl)ethenyl]-ester zu und rührt weitere 2½ Stunden.

Nach dem Abziehen des Lösungsmittels wird zwischen 500 ml Wasser und 400 ml Diethylether verteilt, noch einmal mit 200 ml Diethylether extrahiert und die organische Phase über $Na_2SO_4$/Aktivkohle getrocknet, filtriert und auf 400 ml eingeengt. Nun leitet man unter Eiskühlung und kräftiger Rührung eine Stunde trockenen Chlorwasserstoff ein, kühlt auf -20 °C, filtriert das ausgefallene Hydrochlorid ab und digeriert mit 2x100 ml trockenem Diethylether.

Ausbeute: 37.74 g farblose Kristalle (57% d.Th.)
DC (Amin): *LM*...Bz:$Et_2O$ = 5:1; 0.4
Fp.: 134-136 °C (Ether)
$^1$H-NMR: (DMSO-$d_6$)

d(ppm): 7.79 (dd, 1H, Fu-H5); 7.46 ($s_{breit}$, 3H, $NH_3Cl$); 6.90 (dd, 1H, Fu-H3); 6.87 (s, 1H, Th-H4); 6.62 (dd, 1H, Fu-H4); 3.72 (s, 3H, $CH_3$) $^{13}$C-NMR: (DMSO-$d_6$)

d(ppm): 164.0; 154.2; 148.0; 144.1; 136.9, 115.3; 112.7; 108.6; 97.5, 51.3

**3-Chlorsulfonyl-5-(2-furyl)-2-thiophencarbonsäuremethylester**

Zu einer Suspension von 35.42 g (136 mMol) 3-Amino-5-(2-furyl)-2-thiophencarbonsäuremethylesterhydrochlorid in 215 ml conz. Salzsäure werden während ½ Stunde bei -12 °C 11.29 g (164 mMol) Natriumnitrit in 16 ml Wasser unter die Flüssigkeitsoberfläche eingebracht und eine Stunde gut gerührt.

Parallel dazu versetzt man unter Eiskühlung in einem hohen Becherglas 725 ml einer bei 0 °C gesättigten Schwefeldioxidlösung in Eisessig (~ 40%ig) mit 47 ml einer bei Raumtemperatur gesättigten wäßrigen Kupfer(II)chloridlösung. Zu dieser Mischung wird die Suspension des Diazoniumsalzes aufeinmal zugegeben und auf 30 °C erwärmt. Den dadurch verursachten Volumsverlust gleicht man unmittelbar mit 70 ml der Schwefeldioxidlösung aus und rührt insgesamt 2 Stunden bei Raumtemperatur nach.

Das Reaktionsgemisch wird auf 2 l Eiswasser gegossen, die entstandene Fällung abfiltriert und dreimal mit je 300 ml kaltem Wasser digeriert. Den Feststoff verteilt man zwischen 400 ml Wasser und 300 ml Dichlormethan und extrahiert noch dreimal mit je 200 ml Dichlormethan. Die organische Phase wird über $Na_2SO_4$/Aktivkohle getrocknet, filtriert und das Lösungsmittel abgezogen.

Ausbeute: 38.46 g braune Kristalle (92% d.Th.)
DC: *LM*...Bz:$Et_2O$ = 10:1; 0.7
Fp.: 133-136 °C (Zers)
$^1$H-NMR: ($CDCl_3$)

d(ppm): 7.68 (s, 1h, Th-H4); 7.51 (dd. 1H, Fu-H5);6.77 (dd, 1H, Fu-H3); 6.53 (dd, 1H, Fu-H4); 3.99 (s, 3H, $CH_3$)

$^{13}$C-NMR: ($CDCl_3$)
d(ppm): 158.7; 146.1; 144.4; 144.1; 137.5; 131.5; 123.3; 112.5; 109.4; 53.1

**5-(2-Furyl)-3-[N-(methoxycarbonylmethyl)-sulfamoyl]-2-thiophencarbonsäure-methylester**

Zu einer Suspension von 30.57 g (99.7 mMol) 3-Chlorsulfonyl-5-(2-furyl)-2-thiophencarbonsäuremethylester und 15.64 g (125 mMol) Glycinmethylesterhydrochlorid in 255 ml absolutem Methanol werden bei 0 °C 22.69 g (224 mMol) Triethylamin getropft und eine Stunde gerührt.

Das Reaktionsgemisch wird auf 750 ml eiskalte 2n Salzsäure gegossen, auf etwa -15 °C gekühlt, das entstandene Kristallisat abfiltriert, sowie dreimal mit je 200 ml eiskaltem Wasser digeriert. Das Rohprodukt

wird aus Methanol/Aktivkohle umkristallisiert.
Ausbeute: 29.61 g braune Kristalle (83% d.Th.)
DC: *LM*.. .Bz:$Et_2O$ = 10:1; 0.3
Fp.: 102-105 °C (Methanol) $^1$H-NMR: ($CDCl_3$)
d(ppm): 7.60 (s, 1 H, Th-H4); 7.48 (dd, 1 H, Fu-H5); 6.91 (t, 1H, NH); 6.73 (dd, 1H, Fu- H3); 6.51 (dd, 1H, Fu-H4); 3.95 (s, 3H, Th-$COOCH_3$); 3.92 (d, 2H, $CH_2$); 3.62 (s, 3H, $CH_2$-$COOCH_3$)
$^{13}$C-NMR: ($CDCl_3$)
d(ppm): 168.9; 160.9; 146.8; 145.0; 143.7; 137.6; 127.5; 124.3; 112.3; 108.9; 53.0; 52.3; 44.5

**6-(2-Furyl)-2H-thieno[2,3-e]-1,2-thiazin-3-carbonsäuremethylester- 1,1-dioxid**

Zu 23.06 g (206 mMol) Kalium-tert.-butanolat in 265 ml absolutem Tetrahydrofuran tropft man bei -10 bis -5 °C 32.11 g (89.3 mMol) 5-(2-Furyl)-3-[N-(methoxycarbonylmethyl)-sulfamoyl]-2-thiophencarbonsäuremethylester in 330 ml absolutem Tetrahydrofuran. Das Reaktionsgemisch $\frac{1}{2}$ Stunde gerührt, auf einmal mit 1.2 l eiskalter 2n Salzsäure versetzt. Man filtriert das Rohprodukt ab, digeriert dreimal mit je 250 ml kaltem Wasser und einmal mit 100 ml kaltem Methanol.
Ausbeute: 23.66g orangebraune Kristalle (81% d.Th.)
DC: *LM*...$CH_2Cl_2$:MeOH = 40:1; 0.4
Fp.: 215-222 °C (Zers., Methanol)
$^1$H-NMR: (DMSO-$d_6$)
d(ppm): 7.88 (s, 1H, TT-H7); 7.87 (d, 1H, Fu-H5); 7.22 (d, 1H, Fu-H3); 6.70 (dd, 1H, Fu-H4); 3.89 (s, 3H, $CH_3$)
$^{13}$C-NMR: (DMSO-$d_6$)
d(ppm): 166.8; 149.7; 146.8; 144.6; 140.4; 138.6; 132.0; 116.2; 112.9; 109.7; 104.9; 52.7

**6-(2-Furyl)-2-methyl-2H-thieno[2,3-e]-1,2-thiazin-3-carbonsäuremethylester-1,1-dioxid**

Zu einer Suspension von 1.84 g (76.7 mMol) Natriumhydrid in 40 ml absolutem DMF werden bei -10 °C bis -7 °C 22.81 g (69.7 mMol) 6-(2-Furyl)-2H-thieno[2,3-e]-1,2-thiazin-3-carbonsäuremethylester-1,1-dioxid in 160 ml absolutem DMF getropft und eine Stunde nachgerührt. Die erhaltene Lösung wird mit 11.87 g (83.6 mMol) Jodmethan versetzt und 20 Stunden bei Raumtemperatur gerührt. Man hydrolysiert mit 1 l eiskalter 2n Salzsäure. Der entstandene Niederschlag wird abfiltriert, zweimal mit je 100 ml kaltem Wasser und einmal mit 70 ml kaltem Methanol digeriert. Das Rohprodukt aus 250 ml Dioxan/Aktivkohle umkristallisiert und mit 20 ml kaltem Aceton digeriert.
Ausbeute: 19.73 g gelbe Kristalle (83% d.Th.)
DC: *LM*...Bz:MeOH = 10:1; 0.65
Fp.: 219-222 °C (Zers., Dioxan)
$^1$H-NMR: (DMSO-$d_6$)
d(ppm): 7.91 (s, 1H, TT-H7); 7.87 (d, 1H, Fu-H5); 7.26 (d, 1H, Fu-H3); 6.71 (dd, 1H, Fu-H4); 3.85 (s, 3H, $OCH_3$); 2.95 (s, 3H, $NCH_3$);
$^{13}$C-NMR: (DMSO-$d_6$)
d(ppm): 167.0; 153.5; 146.6; 144.8; 139.8; 139.4; 131.3; 117.5; 113.0; 110.1; 109.1; 52.7; 38.4

**Beispiel 4:**

**6-Chlor-N-{4-[5-(4-fluorphenoxy)-2-furanyl]-2-thiazolyl}-4-hydroxy-2-methyl-2H-thieno[2,3-e]-1,2-thiazin-3-carboxamid-1,1-dioxid**

0.446g (1.44 mmol) 6-Chlor-4-hydroxy-2-methyl-2H-thieno[2,3-e]-1,2-thiazin-3-carbon-säuremethylester-1,1-dioxid und 0.404 g (1.46 mmol) 4-[5-(4-Fluorphenoxy)-2-furanyl]-2-thiazolamin werden in 4 ml absolutem Xylol zum Sieden erhitzt. Nach 4.5 Std. wird die Suspension gekühlt, das Produkt abfiltriert, mehrmals mit Diethylether digeriert und aus Toluol umkristallisiert.
Ausbeute: 0.373 g gelbe Kristalle (46.6% d.Th.)
Fp: Zers. ab 220°C ($Et_2O$)
DC: $CH_2Cl_2$/MeOH 5:1 Rf: 0.5
$^1$H-NMR: (DMSO-$d_6$)
$\delta$ (ppm) = 7.87 (d; 1H, Fu-H5); 7.81 (s; 1H, Th-H7); 7.23 (s; 1H, Thiaz-H5); 7.22-7.07 (m; 5H, Ph-H2,3,5,6); 6.80 (d; 1H, Fu-H3, $^3J_{H,H4}$ = 3.3 Hz); 6.65 (m; 1H, Fu-H4); 5.84 (d; 1H, Fu-H4, $^3J_{H,H4}$ = 3.3 Hz); 2.98 (s; 3H,

$-N-CH_3$)
$^{13}C$-NMR: (DMSO-$d_6$)
$\delta$ (ppm) = 164.88 (s; C=O); 160.35 (s; Th-C4); 158.65 (d; Ph-C4, $^1J_{C,F}$ = 240.6 Hz); 155.73 (s; Thiaz-C2, Fu-C5*); 152.09 (d; Ph-Cl, $^4J_{C,F}$ = 2.3 Hz); 141.07 (s; Fu-C2); 137.50 (s; Th-C4a); 137.00 (s; Th-C7a, Thiaz-C4)*; 135.28 (s; Th-C6); 122.85 (d; Th-C7); 118.88 (dd; Ph-C2,6, $^3J_{C,F}$ = 8.7 Hz); 116.69 (dd; Ph-C3,5, $^2J_{C,F}$ = 23.7 Hz); 110.10 (s; Th-C3); 108.54 (d; Fu-C3); 106.08 (s; Thiaz-C5); 91.14 (d; Fu-C4); 39.09 (q; $-N-CH_3$)
Das Ausgangsmaterial kann wie folgt hergestellt werden:

**5-(4-Fluorphenoxy)-2-furancarbonsäure**

10.03 g (48.65 mmol) 5-(4-Fluorphenoxy)-2-furancarbaldehyd werden in 200 ml wäßriger 4n Natronlauge suspendiert und bei 65 °C möglichst rasch eine Lösung aus 18.10 g (106.6 mmol) Silbernitrat in 100 ml dest. Wasser zugesetzt. Nach 90 Min. wird die heiße Lösung über Hyflo filtriert und 3x mit je 40 ml heißer, wäßriger 4n Natronlauge nachgewaschen.
Das Filtrat wird bei 0 °C mit 4n wäßriger Salzsäure auf pH 3 eingestellt und 7x mit je 100 ml Diethylether extrahiert. Die vereinigten organischen Phasen werden bis auf ca. 50 ml eingeengt, und das ausgefallene Produkt in 300ml halbgesättigter Natriumbicarbonatlösung aufgenommen. Die organische Phase wird abgetrennt und die wäßrige Phase 2x mit je 25ml Diethylether extrahiert. Die wäßrige Phase wird bei 0 °C mit konz. Salzsäure auf pH 3 gestellt, 5x mit je 50 ml Diethylether extrahiert, die vereinigten organischen Phasen 2x mit je 20 ml 2n Natronlauge und einmal mit 20 ml Wasser gewaschen. Danach wird über Natriumsulfat getrocknet, abfiltriert und das Lösungsmittel abgezogen.
**Ausbeute:** 7.48g farblose Kristalle (69.2% d.Th.)
**Fp:** Zers. ab 110 °C ($Et_2O$)
**DC:** Bz/EE 8:1 Rf: 0.7
**$^1$H-NMR:** (DMSO-$d_6$)
$\delta$ (ppm) = 7.34-7.25 (m; 4H, Ph-H2,3,5,6); 7.21 (d; 1H, Fu-H4, $^3J_{H,H3}$ = 4 Hz); 5.78 (d; 1H, Fu-H3, $^3J_{H,H4}$ = 4 Hz)
**$^{13}$C-NMR:** (DMSO-$d_6$)
$\delta$ (ppm) = 159.08 (d; Ph-C4, $^1J_{C,F}$ = 241.4 Hz); 158.82 (s; Fu-C2); 158.69 (d, Fu-C4); 150.77 (d, Ph-Cl, $^4J_{C,F}$ = 2.4 Hz); 136.68 (s; Fu-C5); 120.06 (dd; Ph-C2,6, $^3J_{C,F}$ = 8.7 Hz); 116.75 (dd; Ph-C3,5, $^2J_{C,F}$ = 23.8 Hz); 90.17 (d, Fu-C3)

**2-(4-Fluorphenoxy)-furan**

7.45 g (33.53 mmol)5-(4-Fluorphenoxy)-2-furancarbonsäure werden im Kugelrohr bei 38 mbar auf 110 °C erhitzt, wobei das entstehende Produkt in die Vorlage destilliert.
**Ausbeute:** 4.54 g gelbliche Flüssigkeit (76.0 % d.Th.)
**Kp:** 95-100°C/38mbar
**$n_D^{20}$:**1.5159
**DC:** Bz/EE 8:1 Rf:0.7
**$^1$H-NMR:** (DMSO-$d_6$)
$\delta$ (ppm) = 7.32 (d; 1H, Fu-H5, $^3J_{H,H4}$ = 2 Hz); 7.25-7.05 (m; 4H, Ph-H2,3,5,6); 6.46 (dd; 1H, Fu-H4, $^3J_{H,H3}$ = 4 Hz, $^3J_{H,H5}$ = 2 Hz); 5.74 (d; 1H, Fu-H3, $^3J_{H,H3}$ = 4 Hz)
**$^{13}$C-NMR:** (DMSO-$d_6$)
$\delta$ (ppm) = 158.40 (d; Ph-C4, $^1J_{C,F}$ = 240.0 Hz); 155.87 (s; Fu-C2); 152.40 (d, Ph-C1, $^4J_{C,F}$ = 2.3 Hz); 135.79 (d; Fu-C5); 118.84 (dd; Ph-C2,6, $^3J_{C,F}$ = 8.6 Hz); 116.45 (dd; Ph-C3,5, $^2J_{C,F}$ = 23.7 Hz); 111.37 (d; Fu-C4); 89.16 (d; Fu-C3)

**1-[5-(4-Fluorphenoxy)-2-furanyl]-2-chlorethanon**

Zu 5.18 g (29.08 mmol) 2-(4-Fluorphenoxy)-furan in 40 ml absolutem Tetrahydrofuran werden bei -75 °C 1.86g (29.08 mmol) n-Butyllithium (2.5 n in n-Hexan) getropft und 2.5 Std. nachgerührt. Dann werden 6.14 g (50.51 mmol) 2-Chlor-N,N-dimethylacetamid in 30 ml absolutem Tetrahydrofuran bei - 75 °C zugesetzt.
Nach 60 Min. wird auf 200 ml Eiswasser gegossen, bei 0 °C mit 2n Salzsäure neutralisiert und 5x mit je 40 ml Essigsäureethylester extrahiert. Die vereinigten organischen Phasen werden 2x mit je 20 ml Wasser gewaschen, über Natriumsulfat getrocknet, abfiltriert und das Lösungsmittel abgezogen. Das Produkt wird mittels Säulenchromatographie (360 g KG 60; LM: Toluol) gereinigt und aus Diisopropylether umkristallisiert.
**Ausbeute:** 3.0 g farblose Kristalle (41.1 % d.Th.)
**Fp:** 99-100 °C (DIPE)
**DC:** Bz/EE 8:1 Rf: 0.7
**$^1$H-NMR:** ($CDCl_3$)
δ (ppm) = 7.31 (d; 1H, Fu-H4, $^3J_{H,H3}$ = 3.8 Hz); 7,21-7.02 (m; 4H, Ph-H2,3,5,6); 5.51 (d; 1H, Fu-H3, $^3J_{H,H4}$ = 3.8 Hz); 4.45 (s; 2H, -C$\underline{H}_2$-Cl)
**$^{13}$C-NMR:** ($CDCl_3$)
δ (ppm) = 178.32 (s; C=O); 161.63 (s; Fu-C5); 159.95 (d; Ph-C4, $^1J_{C,F}$ = 245.0 Hz); 149.92 (d, Ph-C1, $^4J_{C,F}$ = 3.4 Hz); 142.13 (s; Fu-C2); 122.43 (d; Fu-C4);.120 46 (dd; Ph-C2,6, $^3J_{C,F}$ = 8.5 Hz); 116.66 (dd; Ph-C3,5, $^2J_{C,F}$ = 23.8 Hz); 89.59 (d; Fu-C3); 44.17 (t; -$\underline{C}H_2$-Cl)

**4-[5-(4-Fluorphenoxy)-2-furanyl]-2-thiazolamin**

2.46 g (9.65 mmol) 1-[5-(4-Fluorphenoxy)-2-furanyl]-2-chlorethanon, 0.758 g (10.00 mmol) Thioharnstoff und 1.33 g (9.62 mmol) Kaliumkarbonat werden in 25 ml absolutem Ethanol zum Sieden erhitzt. Nach 60 Min. wird die abgekühlte Lösung auf 150 ml Wasser gegossen und 5x mit je 40 ml Essigsäureethylester extrahiert. Die vereinigten organischen Phasen werden einmal mit 20 ml Wasser gewaschen, über Natriumsulfat getrocknet, abfiltriert und das Lösungsmittel abgezogen.
Das Produkt wird aus Diisopropylether umkristallisiert.
**Ausbeute:** 1.46g farblose Kristalle (54.7 % d.Th.)
**Fp:** 108-110 °C (DIPE)
**DC:**
EE Rf: 0.8
Bz/EE 8:1 Rf: 0.1
**$^1$H-NMR:** (DMSO-$d_6$)
δ (ppm) = 7.32-7.08 (m; 4H, Ph-H2,3,5,6); 6.60 (s; 1H, Thiaz-H5); 6.52 (d; 1H, Fu-H3, $^3J_{H,H4}$ = 3.3 Hz); 5.78 (d; 1H, Fu-H4, $^3J_{H,H3}$ = 3.3 Hz)
**$^{13}$C-NMR:** (DMSO-$d_6$)
δ (ppm) = 168.75 (s; Thiaz-C2); 158.57 (d; Ph-C4, $^1J_{C,F}$ = 240.3 Hz); 154.98 (s; Fu-C5); 152.31 (d; Ph-C1, $^4J_{C,F}$ = 2.3 Hz); 143.34 (s; Fu-C2); 140.99 (s; Thiaz-C4); 118.67 (dd; Ph-C2,6, $^3J_{C,F}$ = 8.6 Hz); 116.61 (dd; Ph-C3,5, $^2J_{C,F}$ = 23.7 Hz); 107.09 (d; Fu-C3); 99.75 (d; Thiaz-C5); 90.94 (d; Fu-C4)

**Beispiel 5**

**N-{4-[5-(4-Fluorphenoxy)-2-furanyl]-2-thiazolyl}-6-(2-furanyl)-4-hydroxy-2-methyl-2H-thieno[2,3-e]-1,2-thiazin-3-carboxamid-1,1-dioxid**

0.339 g (0.99 mmol) 6-(2-Furanyl)-4-hydroxy-2-methyl-2H-thieno[2,3-e]-1,2-thiazin-3-carbonsäuremethylester-1,1-dioxid und 0.398 g (1.44 mmol) 4-[5-(4-Fluorphenoxy)-2-furanyl]-2-thiazolamin werden in 3 ml absolutem Xylol zum Sieden erhitzt.
Nach 4.5 Std. wird die Suspension gekühlt, das ausgefallene Produkt abfiltriert, mehrmals mit Diethylether digeriert und aus Dimethylsulfoxid/Aceton 10:1 umkristallisiert.
**Ausbeute:** 0.30 g gelbe Kristalle (51.7 % d.Th.)
**Fp**: Zers. ab 243 °C (DMSO)
**DC:** $CH_2Cl_2$/MeOH 5:1 Rf:0.8
**$^1$H-NMR:** (DMSO-d6)
δ (ppm) = 7.82 (m; 1H, Fu-H5); 7.81 (s; 1H, Th-H7); 7.23 (s; 1H, Thiaz-H5); 7.22-7.07 (m; 3H, Ph-H2,3,5,6, Fu-H3); 6.80 (d; 1H, ThiazFu-H3, $^2J_{H,H4}$ = 3.3 Hz); 6.65 (m; 1H, Fu-H4); 5.84 (d; 1H, ThiazFu-H4, $^2J_{H,H3}$ = 3.4 Hz); 2.98 (s; 3H, -N-C$\underline{H}_3$)
**$^{13}$C-NMR**: (DMSO-$d_6$) δ (ppm) = 164.99 (s; C=O); 159.01 (s; Thiaz-C2); 158.63 (d; Ph-C4, $^1J_{C,F}$ = 240.3 Hz); 157.22 (s; ThiazFu-C5); 155.61 (s; Th-C4); 152.11 (s; Ph-C1); 147.01 (s; Fu-C2); 144.43 (s; Fu-C5); 141.51 (s; ThiazFu-C2); 138.56 (s; Th-C7a*); 138.43 (s; Thiaz-C4*); 137.67 (s; Th-C4a); 135.18 (s; Th-C6*); 118.85 (dd; Ph-C2,6, $^3J_{C,F}$ = 8.7 Hz); 117.57 (d; Th-C7); 116.68 (dd; Ph-C3,5, $^2J_{C,F}$ = 23.7 Hz); 112.90 (s; Fu-C3); 109.75 (S; Th-C3); 109.31 (d; Fu-C4*); 108.32 (d; ThiazFu-C3*); 106.14 (d; Thiaz-C5); 91.17 (d; ThiazFu-C4); 39.08 (q; -N-$\underline{C}H_3$)

**Beispiel 6**

**6-Chlor-N-{4-[5-(4-fluorphenyl)-2-furanyl]-2-thiazolyl}-4-hydroxy-2-methyl-2H-thieno[2,3-e]-1,2-thiazin-3-carboxamid-1,1-dioxid**

0.209 g (0.68 mmol) 6-Chor-4-hydroxy-2-methyl-2H-thieno[2,3-e]-1,2-thiazin-3-carbon-säuremethylester-1,1-dioxid und 0.182 g (0.70 mmol) 4-[5-(4-Fluorphenyl)-2-furanyl]-2-thiazolamin werden in 2.5 ml absolutem Xylol 5 Std. zum Sieden erhitzt. Nach dem Abkühlen wird das ausgefallene Produkt abfiltriert, 3x mit Diethylether digeriert und aus Dimethylsulfoxid umkristallisiert.
**Ausbeute:** 0.320 g gelbe Kristalle (88.2 % d.Th.)
**Fp**: Zers. ab 250 °C (DMSO)
**DC:**
EE Rf:0.4
$CH_2Cl_2$/MeOH 9:1 Rf: 0.2
**$^1$H-NMR:** (DMSO-$d_6$)
δ (ppm) = 7.88 (dd; 2H, Ph-H2,6, $^3J_{H,H3(5)}$ = 8.4 Hz, $^3J_{H,F}$ = 5.6 Hz); 7.68 (S; 1 H, Th-H7); 7.51 (S; 1 H, Thiaz-H5); 7.35 (dd; 2H, Ph-H3,5, $^3J_{H,H2(6)}$ = $^3J_{H,F}$ = 8.8 Hz); 7.08 (d; 1H, Fu-H3, $^3J_{H,H4}$ = 3.3 Hz); 6.89 (d; 1H, Fu-H4, $^3J_{H,H3}$ = 3.3 Hz); 2.96 (s; 3H, -N-C$\underline{H}_3$)
**$^{13}$C-NMR:** (DMSO-$d_6$)
δ(ppm) = 164.85 (s; C=O); 161.66 (d; Ph-C4, $^1J_{C,F}$ = 245.2 Hz); 160.46 (s; Th-C4); 156.72 (s; Thiaz-C2); 152.00 (s; Fu-C5); 147.91 (s; Fu-C5); 138.08 (s; Thiaz-C4); 137.33 (s; Th-C4a); 136.92 (s; Th-C7a); 135.14 (s; Th-C6); 126.54 (d; Ph-C1, $^4J_{C,F}$ = 3.1 Hz); 125.74 (dd; Ph-C2,6, $^3J_{C,F}$ = 8.3 Hz); 122.81 (d; Th-C7); 115.95 (dd; Ph-C3,5, $^2J_{C,F}$ = 22.0 Hz); 109.97 (s; Th-C3); 109.38 (d; Fu-C3); 107.68 (d; Thiaz-C5*); 107.18 (d; Fu-C4*); 39.09 (q; -N-$CH_3$)
Das Ausgangsmaterial kann wie folgt hergestellt werden:

**1-[5-(4-Fluornhenyl)-2-furanyl]-2-chlorethanon**

Zu 4.44 g (27.41 mmol) 2-(4-Fluorphenyl)-furan in 40 ml absolutem Tetrahydrofuran werden bei -75 °C 1.60 g (21.98 mmol) n-Butyllithium (2.5 n in n-Hexan) getropft. Nach 45 Min. nachrühren werden 5.21 g (42.86 mmol) 2-Chlor-N,N-dimethylacetamid in 20 ml absolutem Tetrahydrofuran bei -75 °C zugesetzt. Nach 60 Min. wird auf 200 ml Eiswasser gegossen, bei 0 °C mit 2n Salzsäure neutralisiert, 6x mit je 60 ml Essigsäureethylester extrahiert, die vereinigten organischer) Phasen 2x mit je 20 ml Wasser gewaschen, über Natriumsulfat getrocknet, abfiltriert und das Lösungsmittel abgezogen.

Das Produkt wird mittels Säulenchromatographie (280 g KG 60; LM: PE/EE 20:1) gereinigt, aus Ethanol umkristallisiert und mit Diisopropylether digeriert.
**Ausbeute:** 2.3 g farblose Kristalle (35.2 % d.Th.)
**Fp**: 108-110°C (DIPE)
**DC:**
Bz/EE 8:1 Rf:0.6
PE/EE20:1 Rf:0.1
**$^1$H-NMR**: ($CDCl_3$)
δ (ppm) = 7.77 (dd; 2H, Ph-H2,6, $^3J_{H,H3(5)}$ = 8.8 Hz, $^3J_{H,F}$ = 5.3 Hz); 7.40 (d; 1H, Fu-H3, $^3J_{H,H4}$ = 3.8 Hz); 7.14 (dd; 2H, Ph-H3,5, $^3J_{H,H2(6)}$ = $^3J_{H,F}$ = 8.6 Hz); 6.77 (d; 1H, Fu-H4, $^3J_{H,H3}$ = 3.8 Hz) 4.58 (s; 2H, -C$\underline{H}_2$-Cl)
**$^{13}$C-NMR:** ($CDCl_3$)
δ (ppm) = 197.48 (s; C=O); 163.30 (d; Ph-C4, $^1J_{C,F}$ = 250.7 Hz); 157.57 (d; Fu-C3); 149.32 (d; Fu-C4); 127.01 (dd; Ph-C2,6, $^3J_{C,F}$ = 8.5 Hz); 125.17 (d, Ph-Cl, $^4J_{C,F}$ = 3.4 Hz); 121.16 (s; Fu-C5); 116.05 (dd; Ph-C3,5, $^2J_{C,F}$ = 21.2 Hz); 107.46 (s; Fu-C2); 44.80 (t; -$\underline{C}H_2$-Cl)

**4-[5-(4-Fluorphenyl)-2-furanyl]-2-thiazolamin**

1.55 g (6.50 mmol) 1-[5-(4-Fluorphenyl)-2-furanyl]-2-chlorethanon, 0.517 g (6.79 mmol) Thioharnstoff und 0.925 g (6.70 mmol) Kaliumcarbonat werden in 7 ml absolutem Ethanol zum Sieden erhitzt. Nach 60 Min. wird die Suspension auf 150 ml Wasser gegossen, 5x mit je 40 ml Essigsäureethylester extrahiert, die vereinigten organischen Phasen 2x mit je 20 ml Wasser gewaschen, über Natriumsulfat getrocknet, abfiltriert und das Lösungsmittel abgezogen. Das Produkt wird mit Diisopropylether digeriert.
**Ausbeute:** 1.26 g farblose Kristalle (74.6 % d.Th.)

**Fp**: 172-173 °C (DIPE)
**DC:**
EE Rf: 0.8
$CH_2Cl_2$/EE 5:1 Rf: 0.3
**$^1$H-NMR:** (DMSO-$d_6$)
δ (ppm) = 7.85-7.71 (dd; 2H, Ph-H2,6, $^3J_{H,H3,(5)}$ = 8.9 Hz, $^3J_{H,F}$ = 5.4 Hz); 7.26 (dd; 2H, Ph-H3,5, $^3J_{H,H2(6)}$ = $^3J_{H,F}$ = 8.9 Hz); 6.97 (d; 1H, Fu-H3, $^3J_{H,H4}$ = 3.4 Hz); 6.90 (s; 1H, Thiaz-H5); 6.62 (d; 1H, Fu-H4,$^3J_{H,H3}$ = 3.4 Hz)
**$^{13}$C-NMR:** (DMSO-$d_6$)
δ (ppm) = 168.83 (s; Thiaz-C2); 159.05 (d; Ph-C4, $^1J_{C,F}$ = 250.0 Hz); 151.16 (s; Fu-C5*); 150.19 (s; Fu-C2*); 141.55 (s; Thiaz-C4); 126.87 (s; Ph-C1); 125.45 (dd; Ph-C2,6, $^3J_{C,F}$ = 8.2 Hz); 115.87 (dd; Ph-C3,5, $^2J_{C,F}$ = 22.0 Hz); 108.23 (d; Fu-C3*); 107.43 (d; Thiaz-C5*); 101.11 (d; Fu-C4)

**Beispiel 7**

**N-{4-[5-(4-Fluorphenyl)-2-furanyl]-2-thiazolyl}-6-(2-furanyl)-4-hydroxy-2-methyl-2H-thieno[2,3-e]-1,2-thiazin-3-carhoxamid-1,1-dioxid**

0.204 g (0.60 mmol) 6-(2-Furanyl)-4-hydroxy-2-methyl-2H-thieno[2,3-e]-1,2-thiazin-3-carbonsäuremethylester-1,1-dioxid und 0.160 g (0.62 mmol) 4-[5-(4-Fluorphenyl)-2-furanyl]-2-thiazolamin werden in 2.5 ml absolutem Xylol 5 Std. zum Sieden erhitzt. Nach dem Abkühlen wird das ausgefallene Produkt abfiltriert, 3x mit Diethylether digeriert und aus Dimethylsulfoxid umkristallisiert.
**Ausbeute:** 0.246 g orange Kristalle (72.4 % d.Th.)
**Fp:** Zers. ab 250°C (DMSO)
**DC:**
EE Rf: 0.3
$CH_2Cl_2$/MeOH 9:1 Rf: 0.3
**$^1$H-NMR:** (DMSO-$d_6$)
δ (ppm) = 7.96-7.75 (m; 4H, Fu-H5, Th-H5, Ph-H3,5); 7.53 (s; 1H, Thiaz-H5); 7.31 (dd; 2H, Ph-H2,6, $^3J_{H,H3(5)}$ = 3.3 Hz); 7.21 (d; 1H, Fu-H3, $^3J_{H,H4}$ = 3.3 Hz); 7.07 (d; 1H, ThiazFu-H3, $^2J_{H,H4}$ = 3.3 Hz); 6.91 (d; 1H, ThiazFu-H4, $^2J_{H,H3}$ = 3.3 Hz); 6.71 (m; 1H, Fu-H4); 2.98 (s; 3H, -N-C$\underline{H}_3$)
**$^{13}$C-NMR:** (DMSO-$d_6$)
δ (ppm) = 165.54 (s; C=O); 161.63 (d; Ph-64, $^1J_{C,F}$ = 245.1 Hz); 160.28 (s; Thiaz-C2); 156.19 (s; Th-C4); 152.02 (s; ThiazFu-C5); 147.90 (s; ThiazFu-C2); 146.93 (s; Fu-C2); 144.50 (d; Fu-C5); 138.77 (s; Th-C7a); 137.46 (s; Th-C4a*, s; Thiaz-C4*); 134.30 (s; Th-C6); 126.50 (s; Ph-C1); 125.72 (dd; Ph-C2,6, $^3J_{C,F}$ = 7.9 Hz); 117.59 (d; Th-C7); 115.92 (dd; Ph-C3,5, $^2J_{C,F}$ = 22.1 Hz); 112.91 (d; Fu-C3); 110.24 (s; Th-C3); 109.37 (s; Fu-C4*, d; ThiazFu-C3*); 107.67 (d; ThiazFu-C4*, d; Thiaz-C5*); 39.08 (q; -N-$\underline{C}H_3$)

Beispiel 8

**N-[4-(2-Benzo[b]furanyl)-2-thiazolyl]-6-chlor-4-hydroxy-2-methyl-2H-thieno[2,3-e]-1,2-thiazin-3-carboxamid-1,1-dioxid**

0.212 g (0.68 mmol) 6-Chlor-4-hydroxy-2-methyl-2H-thieno[2,3-e]-1,2-thiazin-3-carbonsäure-methylester-1,1-dioxid und 0.154 g (0.71 mmol) 4-(2-Benzo[b]furanyl)-2-thiazolamin werden in 3 ml absolutem Xylol 3.5 Std. zum Sieden erhitzt. Danach wird abgekühlt, das ausgefallene Produkt abfiltriert, mit wenig kaltem Diethylether und mit heißem Ethanol digeriert.
**Ausbeute**: 0.240 g gelbe Kristalle (70.6 % d.Th.)
**Fp**: Zers. ab 225 °C (EtOH)
**DC**: $CH_2Cl_2$/MeOH 9:1 Rf: 0.3
**$^1$H-NMR**: (DMSO-$d_6$)
δ (ppm) = 7.75-7.56 (m; 2H, Bz-H4,7); 7.66 (s; 1H, Thiaz-H5); 7.65 (s; 1H, Th-H7); 7.4-7.26 (m; 2H, Bz-H5,6); 7.23 (s; Bz-H3); 2.94 (s; 3H, -N-C$\underline{H}_3$)
**$^{13}$C-NMR:** (DMSO-$d_6$)
δ (ppm) = 164.92 (s; C=O); 159.60 (s; Thiaz-C2); 155.28 (s; Th-C4); 154.08 (s; Bz-C2); 150.42 (s; Bz-C7a); 138.06 (s; Th-C4a); 137.23 (s; Th-C7a); 136.05 (s; Thiaz-C4*); 135.86 (s; Th-C6*); 128.24 (s; Bz-C4a); 124.82 (d; Bz-C5); 123.33 (d; Bz-C4); 122.86 (d; Th-C7); 121.53 (d; Bz-C6); 111.00 (d; Bz-C7); 110.59 (d; Thiaz-C5*); 110.13 (s; Th-C3*); 102.99 (d; Bz-C3); 39.19 (q; -N-$\underline{C}H_3$)

Beispiel 9

**N-[4-(2-Benzo[b]furanyl)-2-thiazolyl]-6-(2-furanyl)-4-hydroxy-2-methyl-2H-thieno[2,3-e]-1,2-thiazin-3-carboxamid-1,1-dioxid**

0.285g (0.84 mmol) 6-(2-Furanyl)-4-hydroxy-2-methyl-2H-thieno[2,3-e]-1,2-thiazin-3-carbon säuremethylester-1,1-dioxid und 0.194 g (0.90 mmol) 4-(2-Benzo[b]furanyl)-2-thiazolamin werden in 3 ml absolutem Xylol 5 Std. zum Sieden erhitzt. Nach dem Abkühlen wird das ausgefallene Produkt abfiltriert, mit wenig kaltem Diethylether und mit heißem Ethanol digeriert.
**Ausbeute**: 0.340 g gelbe Kristalle (77.3 % d.Th.)
**Fp:** Zers. ab 233 °C (EtOH)
**DC:** $CH_2Cl_2$/MeOH 9:1 Rf: 0.3
**$^1$H-NMR:** (DMSO-$d_6$)
δ (ppm) = 7.83 (s; 2H, Th-H7, Fu-H5); 7.7 (s; 1H; Thiaz-H5); 7.77-7.53 (m; 2H, Bz-H4,7); 7.43-7.13 (m; 3H; Bz-H5,6, Fu-H3); 7.24 (s; 1H, Bz-H3); 6.70 (dd; 1H, Fu-H4); 3.00 (s; 3H, -N-$C\underline{H}_3$)
**$^{13}$C-NMR:** (DMSO-$d_6$)
δ (ppm) = 165.35 (s; C=O); 159.00 (s; Thiaz-C2*); 155.52 (s; Th-C4); 154.08 (s; Bz-C2); 150.57 (s; Bz-C7a); 146.76 (s; Fu-C2); 144.53 (d; Fu-C5); 139.24 (d; Thiaz-C4*); 139.00 (s; Th-C7a*); 138.75 (s; Th-C4a*); 133.06 (s; Th-C6); 128.25 (s; Bz-C4a); 124.88 (d; Bz-C5); 123.30 (d; Bz-C4); 121.51 (d; Bz-C6); 117.62 (d; Th-C7); 112.91 (d; Fu-C3); 110.99 (d; Bz-C7*); 109.98 (d; Fu-C4*); 109.69 (s; Th-C3); 107.85 (d; Thiaz-C5*); 102.95 (s; Bz-C3); 39.35 (q; -N-$\underline{C}H_3$)

**Beispiel 10**

**N-[4-(2-Benzo[b]furanyl)-2-oxazolyl]-6-chlor-4-hydroxy-2-methyl-2H-thieno-[2,3-e]-1,2-thiazin-3-carboxamid-1,1-dioxid**

279 mg (1.39 mmol) 4-(2-Benzo[b]furanyl)-2-oxazolamin und 431 mg (1.39 mmol) 6-Chlor-4-hydroxy-2-methyl-2H-thieno[2,3-e]-1,2-thiazin-3-carbonsäuremethylester-1,1-di-oxid werden in 30 ml abs. Xylol 25 Stunden zum Sieden erhitzt. Nach dem Abkühlen werden die ausgefallenen, orangen Kristalle abfiltriert und 3 mal mit kaltem Diethylether digeriert.
Ausbeute: 240 mg gelbe Kristalle (36 % d. Th.)
**Fp:** 218 °C
**$R_F$** = 0.35 ($CHCl_3$/MeOH = 10/1)
**$^1$H-NMR** (DMSO-$d_6$):
δ(ppm): 8.48 (s, 1H, Thiaz-H7); 7.76 (s, 1H, Bzfu-H3); 7.69 (d, 1H, Bzfu-H4); 7.61 (d, 1H, Bzfu-H7); 7.43-7.22 (m, 2H, Bzfu-H5, H6); 7.18 (s, 1H, Ox-H5); 2.92 (s, 3H, $-CH_3$)
**$^{13}$C-NMR** (DMSO-$d_6$):
δ(ppm): 164.83 (s, -C=O); 155.66 (s, Ox-C2); 154.04 (s, Ox-C4); 153.35 (s, Thiaz-C3); 147.93 (s, Bzfu-C2); 137.40 (s, Thiaz-C4); 136.19 (s, Thiaz-C4a); 135.63 (s, Thiaz-C7a), 132.91 (d, Bzfu-C3); 131.01 (s, Bzfu-C3a); 128.02 (s, Bzfu-C7a); 124.98 (d, Bzfu-C6); 123.36 (d, Bzfu-C5); 123.00 (d, Thiaz-C7); 121.39 (d, Bzfu-C4); 110.97 (d, Bzfu-C7); 109.95 (s, Thiaz-C6); 103.47 (d, Ox-C5); 39.29 (q, $-\underline{C}H_3$)
Das Ausgangsmaterial kann wie folgt hergestellt werden:

**4-(2-Benzo[b]furanyl)-2-oxazolamin**

5.00 g (25.69 mmol) 1-(2-Benzo[b]furanyl)-2-chlor-ethanon und 7.71 g (128.45 mmol) Harnstoff werden in 20 ml abs. Dimethylformamid 2.5 Stunden bei 90 °C gerührt. Das Reaktionsgemisch wird zwischen 100 ml Wasser und 150 ml Essigsäureethylester verteilt. Die organische Phase wird 3 mal mit insgesamt 150 ml 2 N Salzsäure extrahiert. Die vereinten salzsauren Phasen werden mit Natriumhydroxydplätzchen alkalisiert und 3 mal mit insgesamt 150 ml Essigsäureethylester extrahiert. Die vereinten organischen Phasen werden über Natriumsulfat/ Aktivkohle getrocknet, filtriert und das Lösungsmittel abgezogen. Das Produkt wird säulenchromatographisch isoliert. (Laufmittel: Trichlormethan/Methanol = 40/1 50 g Kieselgel 60 $R_F$ = 0.2)
**Ausbeute**: 390 mg farblose Kristalle (7.5 % d.Th.)
**Fp**: 215 °C Zerstezung (Aceton)
$R_F$ = 0.2 (Trichlormethan/Methanol = 40/1)

**$^1$H-NMR** (DMSO-$d_6$):
δ(ppm): 7.89 (s, 1H, Ox-H5); 7.63 (d, 1H, Bzfu-H4); 7.53 (d, 1H, Bzfu-H7); 7.38-7.10 (m, 2H, Bzfu-H5, H6); 6.96 (s, 1H, Bzfu-H3)
**$^{13}$C-NMR** (DMSO-$d_6$):
δ(ppm): 162.18 (s, Ox-C2); 154.19 (s, Ox-C4); 149.93 (s, Bzfu-C2); 131.54 (s, Bzfu-C3a); 129.00 (d, Bzfu-C3); 128.56 (s, Bzfu-C7a); 124.77 (d, Bzfu-C6); 123.45 (d, Bzfu-C5); 121.32 (d, Bzfu-C4); 111.09 (d, Bzfu-C7); 102.51 (d, Ox-C5)

Beispiel 11

**N-[4-(2-Benzo[b]furanyl)-2-oxazolyl]-6-(2-furanyl)-4-hydroxy-2-methyl-2H-thieno[2,3-e]-1,2-thiazin-3-carboxamid-1,1-dioxid**

133 mg (0.66 mmol) 4-(2-Benzo[b]furanyl)-2-oxazolamin und 227 mg (0.66 mmol) 6-(2-Furanyl)-4-hydroxy-2-methyl-2H-thieno[2,3-e]-1,2-thiazin-3-carbonsäuremethylester-1,1-dioxid werden in 3 ml abs. Xylol 5 Stunden zum Sieden erhitzt. Nach dem Erkalten der Lösung werden die ausgefallenen Kristalle abfiltriert, 3 mal mit Diethylether und 2 mal mit heißem Ethanol digeriert.
**Ausbeute**: 150 mg gelbe Kristalle (44 % d. Th.)
**Fp**: 216 °C (EtOH)
**$R_F$** = 0.35 (Trichlormethan/Methanol 10/1)
**$^1$H-NMR** (DMSO-$d_6$):
δ(ppm): 8.54 (s, 1H, Thiaz-H7); 7.92-7.84 (m, 2H, Ox-H5 u. Fu-H5); 7.71 (d, 1H, Bzfu-H4); 7.62 (d, 1H, Bzfu-H7); 7.42-7.23 (m, 3H, Bzfu-H5,H6 u. Fu-H3); 7.21 (s, 1H, Bzfu-H3); 6.77-6.68 (m, 1H, Fu-H4); 2.98 (s, 3H, $-CH_3$)
**$^{13}$C-NMR** (DMSO-$d_6$):
δ(ppm):165.73 (s, -C=O); 156.44 (s, Ox-C2); 154.09 (s, Thiaz-C3); 153.32 (s, Bzfu-C2); 147.99 (s, Fu-C2); 146.78 (s, Ox-C4); 144.71 (d, Fu-C5); 139.45 (Thiaz-C4)*; 139.22 (s, Thiaz-C4a)*; 133.22 (d, Bzfu-C3)*; 132.89 (s, Thiaz-C7a)*; 131.10 (s, Bzfu-C4a); 128.06 (s, Bzfu-C7a); 124.98 (d, Bzfu-C6); 123.37 (d, Bzfu-C5); 121.82 (d, Bzfu-C4); 117.82 (d, Thiaz-C7); 113.00 (d, Fu-C3); 111.01 (d, Fu-C4); 109.87 (d, Bzfu-C7)*; 109.77 (s, Thiaz-C6)*; 103.48 (d, Ox-C5); 39.39 (q, $-CH_3$)

**Beispiel 12**

**N-[4-(2-Benzo[b]thienyl)-2-oxazolyl]-6-chlor-4-hydroxy-2-methyl-2H-thieno[2,3-e]-1,2-thiazin-3-carboxamid-1,1-dioxid**

214 mg (0.99 mmol) 4-(2-Benzo[b]thienyl)-2-oxazolamin und 306 mg (0.99 mmol) 6-Chlor-4-hydroxy-2-methyl-2H-theino[2,3-e]-1,2-thiazin-3-carbonsäuremethylester-1,1-dioxid werden in 19 ml abs. Xylol 5 Stunden zum Sieden erhitzt. Nach dem Erkalten der Lösung werden die ausgefallenen Kristalle abfiltriert, aus Aceton/Aktivkohle umkristallisiert, filtriert und 2 mal mit heißem Acetonitril digeriert.
**Ausbeute:** 104 mg gelte Kristalle (21 % d. Th.)
**Fp**: 211-212 °C (Acetonitril)
**$R_F$** = 0.3 (Trichlormethan/Methanol 10/1)
**$^1$H-NMR** (DMSO-$d_6$):
δ(ppm): 8.50 (s, 1H, Thiaz-H7); 8.05 (d, 1H, Bzth-H4); 7.87 (d, 1H, Bzth-H7); 7.78 (s, 1H, Bzth-H3); 7.72 (s, 1H, Ox-H5); 7.55-7.28 (m, 2H Bzth-H5, H6)
**$^{13}$C-NMR** (DMSO-$d_6$):
δ(ppm): 165.09 (s, -C=O); 155.82 (s, Ox-C2); 153.01 (s, Thiaz-C3); 139.73 (s, Ox-C4); 138.45 (s, Thiaz-C4a); 137.50 (s, Thiaz-C7a); 136.14 (s, Bzth-C2); 135.36 (s, Thiaz-C4); 134.13 (s, Bzth-C3a); 133.10 (s, Bzth-C7a); 132.36 (d, Bzth-C3); 124.81 (d, Bzth-C6); 124.75 (d, Ox-C5); 123.74 (d, Bzth-C4); 123.12 (d, Bzth-C5); 122.50 (d, Thiaz-C7), 120.55 (d, Bzth-C7); 110.01 (s, Thiaz-C6); 39.34 (q, $-\underline{C}H_3$)
Das Ausgangsmaterial kann wie folgt hergestellt werden:

**4-(2-Benzo[b]thienyl)-2-oxazolamin**

4.50 g (21.36 mmol) 1-(2-Benzo[b]thienyl)-2-chlor-ethanon und 6,41 g (106.8 mmol) Harnstoff werden in 20 ml abs. Dimethylformamid 7 Stunden bei 90 °C gerührt. Das Reaktionsgemisch wird zwischen 100 ml Wasser und 150 ml Essigsäureethylester verteilt. Die organische Phase wird 3 mal mit insgesamt 150 ml 2

N Salzsäure extrahiert. Die vereinten salzsauren Phasen werden mit Natriumhydroxidplätzchen alkalisiert und 3 mal mit insgesamt 150 ml Essigsäureethylester extrahiert. Die vereinten organischen Phasen werden über Natriumsulfat/ Aktivkohle getrocknet, filtriert und das Lösungsmittel abgezogen. Das Produkt wird säulenchromatographisch isoliert. (Laufmittel: Trichlormethan/Methanol = 40/1 50 g Kieselgel 60 $R_F$ = 0.2)
**Ausbeute:** 560 mg farblose Kristalle (12 % d.Th.)
**Fp**: 215 °C Zersetzung (Aceton)
**$R_F$** = 0.2 (Trichlormethan/Methanol = 40/1)
**$^1$H-NMR** (DMSO-$d_6$):
δ(ppm): 7.95 (s, 1H, Bzth-H3); 7.93 (d, 1H, Bzth-H4); 7.79 (d, 1H, Bzfu-H7); 7.58 (s, 1H, Ox-H5) 7.40-7.25 (m, 2H, Bzfu-H5,H6)
**$^{13}$C-NMR** (DMSO-$d_6$):
δ(ppm): 161.54 (s, Ox-C2); 139.88 (s, Ox-C4); 138.14 (s, Bzth-C2); 135.14 (s, Bzth-C3a); 134.20 (d, Bzth-C7a); 127.76 (s, Bzth-C3); 124.54 (d, Bzth-C6); 124.17 (d, Ox-C5); 123.29 (d, Bzth-C4); 122.30 (d, Bzth-C5); 118.89 (d, Bzth-C7)

Beispiel 13

**N-[4-(2-Benzo[b]thienyl)-2-oxazolyl]-6-(2-furanyl)-4-hydroxy-2-methyl-2H-thieno[2,3-e]-1,2-thiazin-3-carboxamid-1,1-dioxid**

306 mg (1.41 mmol) 4-(2-Benzo[b]thienyl)-2-oxazolamin und 482 mg (1.41 mmol) 6-(2-Furanyl)-4-hydroxy-2-methyl-2H-thieno[2,3-e]-1,2-thiazin-3-carbonsäure-methylester-1,1-dioxid werden in 7 ml abs. Xylol 24 Stunden zum Sieden erhitzt. Nach dem Erkalten der Lösung werden die ausgefallenen orangen Kristalle abfiltriert und mit kaltem Diethylether digeriert.
**Ausbeute:** 150 mg gelbe Kristalle (19% d. Th.)
**Fp**: 225 °C (Zers. Diethylether)
**$R_F$** = 0.35 (Trichlormethan/Methanol 10/1)
**$^1$H-NMR** (DMSO-$d_6$):
δ(ppm): 8.42 (s, 1H, Thiaz-H7); 7.99 (d, 1H, Bzth-H4); 7.91-7.78 (m, 2H, Bzth-H7 u. Ox-H5); 7.71-7.65 (m, 2H, Bzth-H3 u. Fu-H3); 7.50-7.27 (m, 2H, Bzth-H5,H6); 7.12 (d, 1H, Fu-H5); 6.72-6.68 (m, 1H, Fu-H4); 2.91 (s, 3H, $-CH_3$) **$^{13}$C-NMR** (DMSO-$d_6$):
δ(ppm):163.35 (s, -C=O); 159.95 (s, Ox-C2); 154.03 (s, Thiaz-C3); 147.21 (s, 2C, Fu-C2 u. Thiaz-C6); 144.17 (d, Fu-C5); 139.79 (s, Ox-C4); 138.39 (s, 2C, Thiaz-C3a, C7a)*; 138.14 (s, Bzth-C2)*; 137.72 (s, Thiaz-C4)*; 134.17 (s, Thiaz-C7a); 133.75 (s, Bzth-C7a); 131.36 (d, Bzth-C3); 124.73 (d, Bzth-C6); 124.59 (d, Ox-C5); 123.63 (d, Bzth-C4); 122.47 (d, Bzth-C5); 120.16 (d, Thiaz-C7), 117.43 (d, Bzth-C7); 112.78 (d, Fu-C3); 108.87 (d, Fu-C4); 39.33 (q, - $\underline{C}H_3$)

**Beispiel 14**

**4-Hydroxy-2-methyl-6-phenyl-N-(2-pyridinyl)-2H-theino[2,3-e]-1,2-thiazin-3-carboxamid, 1,1-dioxid**

900 mg (2.56 mMol) 4-Hydroxy-2-methyl-6-phenyl-2H-thieno[2,3-e]-1,2-thiazin-3-carbonsäuremethylester-1,1-dioxid und 482 mg (5.12 mMol) 2-Pyridinamin werden in 18 ml absolutem Xylol 6½ Stunden zum Sieden erhitzt.
Das erkaltete Reaktionsgemisch wird mit 20 ml Diethylether verdünnt, der Niederschlag abfiltriert und mit 15 ml kaltem Diethylether digeriert. Das Rohprodukt noch aus Dimethylsulfoxid/Aktivkohle umkristallisiert.
Ausbeute: 495 mg gelbe Kristalle (47% d.Th.)
DC: LM...$CH_2Cl_2$:MeOH = 40:1; 0.35
Fp.: 235-238 °C (Zers., Methanol)
$^1$H-NMR: (DMSO-$d_6$)
d(ppm): 8.33 (dd, 1H, Py-H6); 8.17 (ddd, 1H, Py-H4); 7.88 (s, 1H, TT-H7); 7.87-7.78 (m, 2H, Bz-H2,6); 7.73 (d, 1H, PyH3); 7.55-7.37 (m, 3H, Bz-H3,4,5); 7.31 (dd, 1H, Py-H5); 2.93 (s, 3H, $C\underline{H}_3$)
$^{13}$C-NMR: ($CF_3COOD$)
d(ppm): 172.6; 162.8; 159.0; 151.3; 151.1; 143.3; 140.6; 134.8; 134.6; 133.9; 132.8; 129.6; 125.0; 122.3; 120.4; 112.2; 43.0
Das Ausgangsmaterial kann wie folgt hergestellt werden:

**3-Chlorsulfonyl-5-phenyl-2-thiophencarbonsäure, methylester**

Zu einer Suspension von 30.24 g (112 mMol) 3-Amino-5-phenyl-2-thiophencarbonsäure-methylesterhydrochlorid in 175 ml conz. Salzsäure werden während 1½ Stunden bei -3°C 8.61 g (125 mMol) Natriumnitrit in 15 ml Wasser unter die Flüssigkeitsoberfläche eingebracht und 2 Stunden nachgerührt.
Diese Lösung wird zu einem Gemisch aus 790 ml gesättigter Schwefeldioxidlösung in Eisessig (~ 40%ig) und 51 ml einer gesättigten wäßrigen Kupfer(II)chloridlösung gegossen und 2 Stunden bei Raumtemperatur nachgerührt.
Das Reaktionsgemisch wird auf 1.3 l Eiswasser gegossen und mit 700 ml sowie zweimal je 350 ml Dichlormethan extrahiert. Die organische Phase wird viermal mit je 700 ml Wasser gewaschen, über $Na_2SO_4$ / Aktivkohle getrocknet, filtriert und das Lösungsmittel abgezogen. Das erhaltene Produkt kristallisiert man aus Toluol/Aktivkohle um.
Ausbeute: 29.96g hellbeige Kristalle (84% d.Th.)
DC: LM...Bz:$Et_2O$ = 10:1; 0.75 Fp.: 152-154°C (Toluol)
$^1$H-NMR: ($CDCl_3$)
d(ppm): 7.80 (s, 1H, Th-H4); 7.67-7.58 (m, 2H, Bz-H2,6); 7.52-7.41 (m, 3H, Bz-H3,4,5); 4.01 (s, 3H, $C\underline{H}_3$)
$^{13}$C-NMR: (DMSO-$d_6$)
d(ppm): 161.1; 152.9; 145.3; 132.5; 129.6; 129.3; 127.1; 126.9; 126.1; 52.6

**3-[N-(Methoxycarbonylmethyl)-sulfamoyl]-5-phenyl-2-thiophencarbonsäure, methylester**

31.31 g (98.8 mMol) 3-Chlorsulfonyl-5-phenyl-2-thiophencarbonsäuremethylester, 13.65 g (109 mMol) Glycinmethylesterhydrochlorid, 15.03 g (109 mMol) trockenes Kaliumcarbonat sowie 160 ml absolutes Dichlormethan und 50 ml absolutes Methanol werden zusammen 3 Stunden bei 30°C gerührt. Man versetzt mit weiteren 2.73 g (21.7 mMol) Glycinmethylesterhydrochlorid bzw. 3.01 g (21.7 mMol) trockenem Kaliumcarbonat und hält die Bedingungen noch eine Stunde aufrecht.
Nach Abziehen des Lösungsmittels wird der Rückstand in 500 ml Eiswasser suspendiert, der Niederschlag abfiltriert und viermal mit je 100 ml kaltem Wasser digeriert. Das erhaltene Rohprodukt wird aus Aceton/Aktivkohle umkristallisiert. Ausbeute: 30.3 g farblose Kristalle (83% d.Th.)
DC: LM...Bz:$Et_2O$ = 5:1; 0.25
Fp.: 151-152°C (Aceton)
$^1$H-NMR: (DMSO-$d_6$)
d(ppm): 7.95-7.72 (m, 4H, Th-H4, Bz-H2,6, N$\underline{H}$; 7.62-7.40 (m, 3H, Bz-H3,4,5); 3.98 (d, 2H, $C\underline{H}_2$); 3.91 (s, 3H, Th-$COOC\underline{H}_3$); 3.55 (s, 3H, $CH_2$-$COOC\underline{H}_3$)
$^{13}$C-NMR: (DMSO-$d_6$)
d(ppm): 169.6; 159.9; 147.3; 145.4; 131.2; 129.7; 129.4; 129.0; 126.0; 125.7; 53.1; 51.8; 44.2

**4-Hydroxy-6-phenyl-2H-thieno[2,3-e]-1,2-thiazin-3-carbonsäure, methylester, 1,1-dioxid**

Zu 13.96 g (124 mMol) Kalium-tert.-butanolat in 170 ml absolutem Tertrahydrofuran tropft man bei -5 bis 0°C unter kräftiger, mechanischer Rührung 20.89 g (56.5 mMol) 3-[N-(Methoxycarbonylmethyl)-sulfamoyl]-5-phenyl-2-thiophencarbonsäuremethyl-ester in 210 ml abolutem Tetrahydrofuran und rührt eine Stunde nach.
Nach Zusatz von 840 ml eiskalter 2n Salzsäure wird der Niederschlag abfiltriert und zweimal mit je 100 ml kaltem Wasser und zweimal mit je 100 ml kaltem Methanol digeriert. Das erhaltene Rohprodukt wird aus Chlorbenzol/Aktivkohle umkristallisiert
Ausbeute: 12.42 g gelbe Kristalle (65% d.Th.)
DC: LM...$CH_2Cl_2$:MeOH = 40:1; 0.4
Fp.: 219-223°C (Zers., Chlorbenzol)
$^1$H-NMR: (DMSO-$d_6$)
d(ppm): 8.05 (s, 1H, TT-H7); 7.93-7.80 (m, 2H, Bz-H2,6); 7.58-7.39 (m, 3H, Bz-H3,4,5); 3.90 (s, 3H, $C\underline{H}_3$)
$^{13}$C-NMR: (DMSO-$d_6$)
d(ppm): 166.9; 149.9; 149.7; 140.6; 133.0; 131.6; 129.7; 129.4; 126.6; 126.1; 117.7; 105.0; 52.7

**4-Hydroxy-2-methyl-6-phenyl-2H-thieno[2,3-e]-1,2-thiazin-3-carbonsäure, methylester, 1,1-dioxid**

Zu einer Suspension von 0.758 g (31.6 mMol) Natriumhydrid in 16 ml absolutem N,N-Dimethylformamid werden bei 10 °C 9.68 g (28.7 mMol) 4-Hydroxy-6-phenyl-2H-thieno[2,3-e]-1,2-thiazin-3-carbonsäuremethylester-1,1-dioxid in 33 ml absolutem N,N-Dimethylformamid getropft und eine Stunde nachgerührt. Die erhaltene Lösung wird mit 4.87 g (34.4 mMol) Jodmethan versetzt und 20 Stunden bei Raumtemperatur gerührt.
Nach Zusatz von 245 ml eiskalter 2n Salzsäure wird der entstandene Niederschlag abfiltriert, zweimal mit je 50 ml kaltem Wasser und einmal mit 30 ml kaltem Aceton digeriert. Das erhaltene Rohprodukt wird aus Toluol/Aktivkohle umkristallisiert und zweimal mit je 50 ml kaltem Petrolether digeriert.
Ausbeute: 8.71 g gelbe Kristalle (86% d.Th.)
DC: LM...Bz:MeOH = 10:1; 0.7
Fp.: 204-211 °C (Zers., Toluol)
$^1$H-NMR: (DMSO-$d_6$)
d(ppm): 8.05 (s, 1H, TT-H7); 7.95-7.79 (m, 2H, Bz-H2,6); 7.58-7.38 (m, 3H, Bz-H3,4,5); 3.90 (s, 3H, O$\underline{CH_3}$); 2.96 (s, 3H, N$\underline{CH_3}$)
$^{13}$C-NMR: (DMSO-$d_6$)
d(ppm): 167.2; 153.6; 151.2; 139.6; 131.5; 129.8; 129.4; 129.4; 126.2; 119.0; 109.2; 52.7; 38.4

Beispiel 15

**4-Hydroxy-2-methyl-N-(2-pyridinyl)-6-(2-thienyl)-2H-thieno[2,3-e]-1,2-thiazin-3-carboxamid, 1,1-dioxid**

1.20 g (3.36 mMol) 4-Hydroxy-2-methyl-6-(2-thienyl)-2H-thieno[2,3-e]-1,2-thiazin-3-carbonsäuremethylester-1,1-dioxid und 632 mg (6.71 mMol) 2-Pyridinamin werden in 24 ml absolutem Xylol 7 Stunden zum Sieden erhitzt.
Das erkaltete Reaktionsgemisch wird mit 25 ml Diethylether verdünnt, der erhaltene Niederschlag abfiltriert und mit 15 ml kaltem Diethylether digeriert. Das Rohprodukt wird aus Dimethylsulfoxid/Aktivkohle umkristallisiert und zweimal mit je 2 ml kaltem Aceton digeriert.
Ausbeute: 679 mg orange Kristalle (48% d.Th.)
DC: LM...$CH_2Cl_2$:MeOH = 40:1; 0.3
Fp.: 239-242 °C (Zers., DMSO)
$^1$H-NMR: (DMSO-$d_6$)
d(ppm): 8.32 (dd, 1H, Py-H6); 8.17 (ddd, 1H, Py-H4); 7.77-7.56 (m, 4H, Py-H3, TT-H7, Th-H3,5); 7.31 (dd, Py-H5); 7.16 (dd, Th-H4); 2.93 (s,3H, $\underline{CH_3}$)
$^{13}$C-NMR: ($CF_3COOD$)
d(ppm): 172.9; 163.5; 153.3; 151.6; 142.8; 140.8; 137.2; 137.1; 133.9; 132.2; 132.1; 131.1; 125.3; 122.1; 120.5; 111.9; 43.3
Das Ausgangsmaterial kann wie folgt hergestellt werden:

**4-Methylbenzolsulfonsäure, [2-cyano-1-(2-thienyl)ethenyl]-ester**

Zu 37.54 g (248 mMol) b-Oxo-2-thiophenpropannitril und 35.16 g (348 mMol) N-Methylmorpholin in 75 ml absolutem Dichlormethan werden bei 10 bis 15 °C 49.70 g (261 mMol) p-Toluolsulfonsäurechlorid in 75 ml absolutem Dichlormethan getropft und eine Stunde nachgerührt.
Das Lösungsmittel wird abgezogen, der Rückstand zwischen 800 ml Essigsäureethylester und 300 ml 0.5n Salzsäure verteilt, über Hyflo filtriert und die wäßrige Phase noch mit 500 ml Essigsäureethylester extrahiert. Die vereinigten Extrakte werden über $Na_2SO_4$/Aktivkohle getrocknet, filtriert und das Extraktionsmittel abdestilliert. Den erhaltenen Feststoff kristallisiert man aus Toluol/Aktivkohle um
Ausbeute: 40.55 g fahlgelbe Kristalle (53% d.Th.)
DC: LM...Bz:$Et_2O$ = 10:1; 0.6
Fp.: 102-104 °C (Toluol)
$^1$H-NMR: (DMSO-$d_6$)
d(ppm): 7.95-7.80 (m, 3H, Bz-H2,6, Th-H5); 7.57-7.42 (m, 3H, Bz-H3,5, Th-H3); 7.15 (dd, 1H, Th-H4); 6.50 (s, 1H, C$\underline{H}$-CN); 2.45 (s, 3H, C$\underline{H_3}$)
$^{13}$C-NMR: (DMSO-$d_6$)
d(ppm): 155.1; 146.7; 134.0; 132.5; 131.4; 130.9; 130.3; 128.6; 128.3; 114.6; 88.3; 21.2

**3-Amino-5-(2-thienyl)-2-thiophencarbonsäure, methylester**

6.65 g (123 mMol) Natriummethanolat in 350 ml absolutem Methanol werden bei 15-20 °C mit 13.06 g (123 mMol) Thioglycolsäuremethylester versetzt und 20 Minuten bei Raumtemperatur gerührt. Anschließend gibt man in einer Portion 35.79 g (117 mMol) 4-Methylbenzolsulfonsäure,[2-cyano-1-(2-thienyl)ethenyl]-ester zu und rührt eine Stunde nach.

Nach dem Abziehen des Lösungsmittels wird zwischen 400 ml Wasser und 300 ml Diethylether verteilt, noch einmal mit 200 ml Dichlormethan extrahiert und die organische Phase über $Na_2SO_4$/Aktivkohle getrocknet, filtriert und das Lösungsmittel abdestilliert. Das Rohprodukt wird aus Toluol/Aktivkohle umkristallisiert.

Ausbeute: 23.14g gelbe Kristalle (82% d.Th.)

DC: LM...Bz:$Et_2O$ = 5:1; 0.4

Fp.: 103-106 °C (Toluol)

$^1$H-NMR: ($CDCl_3$)

d(ppm): 7.35-7.23 (m, 2H, ThB-H3,5); 7.03 (dd, 1H, ThB-H4); 6.66 (s, 1H, ThA-H4); 5.48 ($s_{breit}$, 2H, $N\underline{H}_2$); 3.87 (s, 3H, $C\underline{H}_3$)

$^{13}$C-NMR: ($CDCl_3$)

d(ppm): 164.7; 154.0; 142.0; 136.4; 127.9; 126.0; 125.1; 115.5; 99.2; 51.1

**3-Chlorsulfonyl-5-(2-thienyl)-3-thiopriencarbonsäure, methylester**

Zu einer Suspension von 23.72 g (99.1 mMol) 3-Amino-5-(2-thienyl)-2-thiophencarbon-säuremethylester in 155 ml conz. Salzsäure werden während $\frac{1}{2}$ Stunde bei -10 bis -3 °C 7.86 g (114 mMol) Natriumnitrit in 12 ml Wasser unter die Flüssigkeitsoberflache eingebracht und eine Stunde nachgerührt.

Diese Lösung wird zu einem Gemisch aus 525 ml gesättigter Schwefeldioxidlösung in Eisessig (~ 40%ig) und 34 ml einer gesättigten wäßrigen Kupfer(II)chloridlösung gegossen und bei 30 °C 2 Stunden nachgerührt.

Das Reaktionsgemisch wird auf 1 l Eiswasser gegossen und dreimal je 300 ml Dichlormethan extrahiert. Die organische Phase wird viermal mit je 300 ml Wasser gewaschen, über $Na_2SO_4$/Aktivkohle getrocknet, filtriert und das Lösungsmittel abgezogen. Das erhaltene Rohprodukt wird aus Acetonitril/Aktivkohle umkristallisiert.

Ausbeute: 26.63 g braune Kristalle (83% d.Th.)

DC: LM...Bz:$Et_2O$ = 10:1; 0.8

Fp.: 164-167 °C (Acetonitril)

$^1$H-NMR: (DMSO-$d_6$)

d(ppm): 7.60 (dd, 1H, ThB-H5); 7.44 (dd, 1H, ThB-H3); 7.37 (s, 1H, ThA-H4); 7.11 (dd, 1H, ThB-H4); 3.75 (s, 3H, $C\underline{H}_3$)

$^{13}$C-NMR: (DMSO-$d_6$)

d(ppm): 160.9; 153.0; 139.0; 135.2; 129.2; 127.9; 127.0; 126.5; 126.2; 52.7

**3-[N-(Methoxycarbonylmethyl)-sulfamoyl]-5-(2-thienyl)-3-thiophencarbon-säuremethylester**

23.75 g (73.6 mMol) 3-Chlorsulfonyl-5-(2-thienyl)-3-thiophencarbonsäuremethyl-ester, 10.16 g (80.9 mMol) Glycinmethylesterhydrochlorid` 11.19 g (80.9 mMol) trockenes Kaliumcarbonat sowie 120 ml absolutes Dichlormethan und 40 ml absolutes Methanol werden zusammen eine Stunde zum Sieden erhitzt. Man versetzt mit weiteren 10.16 g (80.9 mMol) Glycinmethylesterhydrochlorid bzw. 11.19 g (80.9 mMol) trockenem Kaliumcarbonat und hält die Bedingungen noch eine Stunde aufrecht.

Nach Abziehen des Lösungsmittels wird der Rückstand in 450 ml Eiswasser suspendiert, das Rohprodukt abfiltriert und viermal mit je 100 ml kaltem Wasser digeriert und aus Toluol/Aktivkohle umkristallisiert.

Ausbeute: 23.70 g hellbeige Kristalle (86% d.Th.)

DC: LM...Bz:$Et_2O$ = 10:1; 0.35

Fp.: 124-126 °C (Toluo)

$^1$H-NMR: ($CDCl_3$)

d(ppm): 7.57 (s, 1H, ThA-H4); 7.39 (dd, 1H, ThB-H5); 7.32 (dd, 1H, ThB-H3); 7.08 (dd, 1H, ThB-H4); 6.91 (t, 1H, N$\underline{H}$); 3.99 (s, 3H, Th-COOC$\underline{H}_3$); 3.96 (d, 2H, C$\underline{H}_2$); 3.62 (s, 3H, $CH_2$-COOC$\underline{H}_3$) $^{13}$C-NMR: ($CDCl_3$)

d(ppm): 168.9; 160.7; 145.0; 142.0; 134.2; 128.4; 127.6; 127.5; 126.3; 125.6; 53.1; 52.3; 44.5

**4-Hydroxy-6-(2-thienyl)-2H-thieno[2,3-e]-1,2-thiazin-3-carbonsäure, methylester, 1,1-dioxid**

Zu 16.95 g (151 mMol) Kalium-tert.-butanolat in 200 ml absolutem Tetrahydrofuran tropft man bei -15°C unter kräftiger, mechanischer Rührung 24.65 g (65.7 mMol) 3-(N-(Methoxycarbonylmethyl)-sulfamoyl]-5-(2-thienyl)-3-thiophencarbonsäuremethyl-ester in 250 ml abolutem Tetrahydrofuran und rührt eine Stunde nach.

Nach Zusatz von 900 ml eiskalter 2n Salzsäure wird der Niederschlag abfiltriert und mit 100 ml kaltem Wasser und zweimal mit je 50 ml kaltem Methanol digeriert. Dieses Rohprodukt wird aus Acetonitril/Aktivkohle umkristallisiert.

Ausbeute: 17.70 g gelbe Kristalle (79% d.Th.)

DC: LM...$CH_2Cl_2$:MeOH = 40:1; 0.4

Fp.: 202-205°C (Zers., Acetonitril)

$^1$H-NMR: (DMSO-$d_6$)

d(ppm): 7.80 (s, 1H, TT-H7); 7.71 (d, 1H, Th-H5); 7.66 (d, 1H, Th-H3); 7.18 (dd, 1H, Th-H4); 3.89 (s, 3H, C<u>H</u>$_3$)

$^{13}$C-NMR: (DMSO-$d_6$)

d(ppm): 166.8; 149.5; 143.1; 140.4; 134.1; 132.2; 128.8; 128.4; 127.2; 117.4; 105.0; 52.7

**4-Hydroxy-2-methyl-6-(2-thienyl)-2H-thieno[2,3-e]-1,2-thiazin-3-carbonsäure, methylester, 1,1-dioxid**

Zu einer Suspension von 1.17 g (48.9 mMol) Natriumhydrid in 65 ml absolutem N,N-Dimethylformamid werden bei 10°C 15.28 g (44.5 mMol) 4-Hydroxy-6-(2-thienyl)-2H-thieno[2,3-e]-1,2-thiazin-3-carbonsäuremethylester-1,1-dioxid in 52 ml absolutem N,N-Dimethylformamid getropft und eine Stunde nachgerührt. Die erhaltene Losung wird mit 7.58 g (53.4 mMol) Jodmethan versetzt und 20 Stunden bei Raumtemperatur gerührt.

Man hydrolysiert mit 600 ml eiskalter 2n Salzsäure, der entstandene Niederschlag wird abfiltriert, zweimal mit je 90 ml kaltem Wasser und einmal mit 30 ml kaltem Aceton digeriert. Das erhaltene Rohprodukt wird aus Dioxan/Aktivkohle umkristallisiert.

Ausbeute: 13.03 g gelbe Kristalle (82% d.Th.)

DC: LM...Bz:MeOH = 10:1; 0.7

Fp.: 212-218°C (Zers., Dioxan)

$^1$H-NMR: (DMSO-$d_6$)

d(ppm): 7.84 (s, 1H, TT-H7); 7.73 (d, 1H, Th-H5); 7.68 (d, 1H, Th-H3); 7.19 (dd, 1H, Th-H4; 3.90 (s, 3H, OC<u>H</u>$_3$); 2.96 (s, 3H, NC<u>H</u>$_3$)

$^{13}$C-NMR: (DMSO-$d_6$)

d(ppm): 167.0; 153.3; 144.3; 139.4; 133.8; 131.5; 128.9; 128.8; 127.5; 118.6; 109.2; 52.7; 38.4

**Beispiel 16**

**6-(2-Furyl)-4-hydroxy-2-methyl-N-(2-pyridinyl)-2H-thieno[2,3-e]-1,2-thiazin-3-carboxamid, 1,1-dioxid**

1.86 g (5.44 mMol) 6-(2-Furyl)-4-hydroxy-2-methyl-2H-thieno[2,3-e]-1,2-thiazin-3-carbonsäuremethylester-1,1-dioxid und 1.02 g (10.9 mMol) 2-Pyridinamin werden in 37 ml absolutern Xylol 4½ Stunden zum Sieden erhitzt.

Das erkaltete Reaktionsgemisch wird mit 50 ml Diethylether verdünnt, der erhaltene Niederschlag abfiltriert und mit 20 ml kaltem Diethylether digeriert. Das Rohprodukt wird aus Dimethylsulfoxid/Aktivkohle umkristallisiert.

Ausbeute: 1.24g gelbe Kristalle (57% d.Th.)

DC: LM...$CH_2Cl_2$:MeOH = 40:1; 0.3

Fp.: 231 -234°C (Zers. DMSO)

$^1$H-NMR: (DMSO-$d_6$)

d(ppm): 8.32 (d, 1H, Py-H6); 8.17 (dd, 1H, Py-H4); 7.82 (d, 1H, Fu-H5); 7.74 (d, 1H, Py-H3); 7.72 (s, 1H, TT-H7); 7.30 (dd, 1H, Py-H5); 7.15 (d, 1H, Fu-H3); 6.68 (dd, 1H, Fu-H4); 2.93 (s, 3H, C<u>H</u>$_3$)

$^{13}$C-NMR: ($CF_3COOD$)

d(ppm): 172.4; 162.7; 151.1; 151.0; 150.0; 148.0; 147.0; 143.5; 140.6; 133.1; 124.8; 120.8 120.3; 115.9; 113.6; 112.1; 42.9

Das Ausgangsmaterial kann wie folgt hergestellt werden:

**4-Methylbenzolsulfonsäure, [2-cyano-1-(2-furyl)ethenyl]-ester**

Zu 45.75 g (339 mMol) b-Oxo-2-furanpropannitril und 47.95 g (474 mMol) N-Methylmorpholin in 100 ml absolutem Dichlormethan werden bei -5 bis 0 °C 67.78 g (356 mMol) p-Toluolsulfonsäurechlorid in 100 ml absolutem Dichlormethan getropft und eine Stunde nachgerührt.
Das Lösungsmittel wird abgezogen, der Rückstand zwischen 500 ml Essigsäureethylester und 400 ml 1n Salzsäure verteilt und die wäßrige Phase mit 6x150 ml Essigsäureethylester extrahiert. Die Vereinten Extrakte werden über $Na_2SO_4$/Aktivkohle getrocknet, filtriert und das Extraktionsmittel abdestilliert. Den erhaltenen Feststoff kristallisiert man aus Toluol/Aktivkohle um.
Ausbeute: 92.48 g farblose Kristalle (94% d.Th.)
DC: *LM*...Bz:$Et_2O$ = 10:1; 0.65
Fp.: 109-111 °C (Toluol)
$^1$H-NMR: (DMSO-$d_6$)
d(ppm): 8.00-7.85 (m, 3H, Bz-H2,6, Fu-H5); 7.60-7.48 (m, 2H, Bz-H3,5) 6.74 (dd, 1H, Fu-H3*); 6.57 (dd, 1H, Fu-H4*); 6.33 (s, 1H, C<u>H</u>-CN); 2.45 (s, 3H, C<u>H</u>$_3$)
$^{13}$C-NMR: (DMSO-$d_6$)
d(ppm): 150.2; 147.7; 146.8; 145.4; 131.1; 130.4; 128.4; 115.9; 114.4; 113.1; 87.3; 21.2

**3-Amino-5-(2-furyl)-2-thiophencarbonsäure,methylester Hydrochlorid**

16.75 g (309 mMol) Natriummethanolat in 750 ml absolutem Methanol werden bei 15-20 °C mit 32.75 g (309 mMol) Thioglycolsäuremethylester versetzt und 20 Minuten bei Raumtemperatur gerührt. Anschließend gibt man in einer Portion 74.38 g (257 mMol) 4-Methylbenzolsulfonsäure-[2-cyano-1-(2-furyl)ethenyl]-ester zu und rührt weitere 2½ Stunden.
Nach dem Abziehen des Lösungsmittels wird zwischen 500 ml Wasser und 400 ml Diethylether verteilt, noch einmal mit 200 ml Diethylether extrahiert und die organische Phase über $Na_2SO_4$/Aktivkohle getrocknet, filtriert und auf 400 ml eingeengt. Nun leitet man unter Eiskühlung und kräftiger Rührung eine Stunde trockenen Chlorwasserstoff ein, kühlt auf -20 °C, filtriert das ausgefallene Hydrochlorid ab und digeriert mit 2x100 ml trockenem Diethylether. Ausbeute: 37.74 g farblose Kristalle (57% d.Th.)
DC (Amin): *LM*...Bz:$Et_2O$ = 5:1; 0.4
Fp.: 134-136 °C (Ether)
$^1$H-NMR: (DMSO-$d_6$)
d(ppm): 7.79 (dd, 1H, Fu-H5); 7.46 ($s_{breit}$, 3H, N<u>H</u>$_3$Cl); 6.90 (dd, 1H, Fu-H3); 6.87 (s, 1H, Th-H4); 6.62 (dd, 1H, Fu-H4); 3.72 (s, 3H, C<u>H</u>$_3$)
$^{13}$C-NMR: (DMSO-$d_6$)
d(ppm): 164.0; 154.2; 148.0; 144.1; 136.9, 115.3; 112.7; 108.6; 97.5, 51.3

**3-Chlorsulfonyl-5-(2-furyl)-2-thiophencarbonsäuremethylester**

Zu einer Suspension von 35.42 g (136 mMol) 3-Amino-5-(2-furyl)-2-thiophencarbonsäuremethylesterhydrochlorid in 215 ml conz. Salzsäure werden während ½ Stunde bei -12 °C 11.29 g (164 mMol) Natriumnitrit in 16 ml Wasser unter die Flüssigkeitsoberfläche eingebracht und eine Stunde nachgerührt.
Diese Lösung wird zu einem Gemisch aus 725 ml gesättigter Schwefeldioxidlösung in Eisessig (~ 40%ig) und 47 ml einer gesättigten wäßrigen Kupfer(II)chloridlösung gegossen, auf 30 °C erwärmt und 2 Stunden nachgerührt.
Das Reaktionsgemisch wird auf 2 l Eiswasser gegossen, die entstandene Fällung abfiltriert und dreimal mit je 300 ml kaltem Wasser digeriert. Den Feststoff verteilt man zwischen 400 ml Wasser und 300 ml Dichlormethan und extrahiert noch dreimal mit je 200 ml Dichlormethan. Die organische Phase wird über $Na_2SO_4$/Aktivkohle getrocknet, filtriert und das Lösungsmittel abgezogen.
Ausbeute: 38.46 g braune Kristalle (92% d.Th.)
DC: *LM*...Bz:$Et_2O$ = 10:1; 0.7
Fp.: 133-136 °C (Zers)
$^1$H-NMR: ($CDCl_3$)
d(ppm): 7.68 (s, 1H, Th-H4); 7.51 (dd, 1H, Fu-H5);6.77 (dd, 1H, Fu-H3); 6.53 (dd, 1H, Fu-H4); 3.99 (s, 3H, C<u>H</u>$_3$)
$^{13}$C-NMR: ($CDCl_3$)
d(ppm): 158.7; 146` 1; 144.4; 144.1; 137.5; 131.5; 123.3; 112.5; 109.4; 53.1

**5-(2-Furyl)-3-[N-(methoxycarbonylmethyl)-sulfamoyl]-2-thiophencarbonsäure-methylester**

Zu einer Suspension von 30.57 g (99.7 mMol) 3-Chlorsulfonyl-5-(2-furyl)-2-thiophencarbonsäuremethylester und 15.64 g (125 mMol) Glycinmethylesterhydrochlorid in 255 ml absolutem Methanol werden bei 0 ° C 22.69 g (224 mMol) Triethylamin getropft und eine Stunde gerührt.
Das Reaktionsgemisch wird auf 750 ml eiskalte 2n Salzsäure gegossen, das enstandene Kristallisat abfiltriert und dreimal mit je 200 ml eiskaltem Wasser digeriert. Das Rohprodukt wird aus Methanol/Aktivkohle umkristallisiert.
Ausbeute: 29.61 g braune Kristalle (83% d.Th.)
DC: *LM*.. .Bz:$Et_2O$ = 10:1; 0.3
Fp.: 102-105 ° C (Methanol)
$^1$H-NMR: ($CDCl_3$)
d(ppm): 7.60 (s, 1H, Th-H4); 7.48 (dd, 1H, Fu-H5); 6.91 (t, 1H, N<u>H</u>; 6.73 (dd, 1H, Fu- H3); 6.51 (dd, 1H, Fu-H4); 3.95 (s, 3H, Th-COOC<u>H</u>$_3$); 3.92 (d, 2H, C<u>H</u>$_2$); 3.62 (s, 3H, $CH_2$-COOC<u>H</u>$_3$)
$^{13}$C-NMR: ($CDCl_3$)
d(ppm): 168.9; 160.9; 146.8; 145.0; 143.7; 137.6; 127.5; 124.3; 112.3; 108.9; 53.0; 52.3; 44.5

**6-(2-Furyl)-4-hydroxy-2H-thieno[2,3-e]-1,2-thiazin-3-carbonsäuremethylester-1,1-dioxid**

Zu 23.06 g (206 mMol) Kalium-tert.-butanolat in 265 ml absolutem Tertrahydrofuran tropft man bei -10 bis -5 ° C 32.11 g (89.3 mMol) 5-(2-Furyl)-3-[N-(methoxycarbonylmethyl)-sulfamoyl]-2-thiophencarbonsäuremethylester in 330 ml absolutem Tetrahydrofuran und rührt eine ½ Stunde nach.
Nach Zusatz von 1.2 l eiskalter 2n Salzsäure wird das Rohprodukt abfiltriert, dreimal mit je 250 ml kaltem Wasser und einmal mit 100 ml kaltem Methanol digeriert.
Ausbeute: 23.66g orange Kristalle (81% d.Th.)
DC: *LM*...$CH_2Cl_2$:MeOH = 40:1; 0.4
Fp.: 215-222 ° C (Zers., Methanol)
$^1$H-NMR: (DMSO-$d_6$)
d(ppm): 7.88 (s, 1H, TT-H7); 7.87 (d, 1H, Fu-H5); 7.22 (d, 1H, Fu-H3); 6.70 (dd, 1H, Fu-H4); 3.89 (s, 3H, C<u>H</u>$_3$)
$^{13}$C-NMR: (DMSO-$d_6$)
d(ppm): 166.8; 149.7; 146.8; 144.6; 140.4; 138.6; 132.0; 116.2; 112.9; 109.7; 104.9; 52.7

**6-(2-Furyl)-4-hydroxy-2-methyl-2H-thieno[2,3-e]-1,2-thiazin-3-carbonsäure-methylester-1,1-dioxid**

Zu einer Suspension von 1.84 g (76.7 mMol) Natriumhydrid in 40 ml absolutem DMF werden bei -10 ° C bis -7 ° C 22.81 g (69.7 mMol) 6-(2-Furyl)-4-hydroxy-2H-thieno[2,3-e]-1,2-thiazin-3-carbonsäuremethylester-1,1-dioxid in 160 ml absolutem DMF getropft und eine Stunde nachgerührt. Die erhaltene Lösung wird mit 11.87 g (83.6 mMol) Jodmethan versetzt und 20 Stunden bei Raumtemperatur gerührt.
Nach Zugabe von 1 l eiskalter 2n Salzsäure wird der entstandene Niederschlag abfiltriert, zweimal mit je 100 ml kaltem Wasser und einmal mit 70 ml kaltem Methanol digeriert. Das Rohprodukt wird aus 250 ml Dioxan/Aktivkohle umkristallisiert und mit 20 ml kaltem Aceton digeriert.
Ausbeute: 19.73 g gelbe Kristalle (83% d.Th.)
DC: *LM*...Bz:MeOH = 10:1; 0.65
Fp.: 219-222 ° C (Zers., Dioxan)
$^1$H-NMR: (DMSO-$d_6$)
d(ppm): 7.91 (s, 1H, TT-H7); 7.87 (d, 1H, Fu-H5); 7.26 (d, 1H, Fu-H3); 6.71 (dd, 1H, Fu-H4); 3.85 (s, 3H, OC<u>H</u>$_3$); 2.95 (s, 3H, NC<u>H</u>$_3$);
$^{13}$C-NMR: (DMSO-$d_6$)
d(ppm): 167.0; 153.5; 146.6; 144.8; 139.8; 139.4; 131.3; 117.5; 113.0; 110.1; 109.1; 52.7; 38.4

**Beispiel 17**

**N-[6-(2-Benzo[b]furyl)-2-pyridinyl]-4-hydroxy-2-methyl-6-phenyl-2H-thieno[2,3-e]-1,2-thiazin-3-carboxamid, 1,1-dioxid**

700 mg (1.99 mMol) 4-Hydroxy-2-methyl-6-phenyl-2H-thieno[2,3-e]-1,2-thiazin-3-carbonsäuremethylester-1,1-dioxid und 419 mg (1.99 mMol) 6-(2-Benzo[b]furyl)-2-pyridinamin werden in 7 ml absolutem Xylol

10 Stunden zum Sieden erhitzt.
Das erkaltete Reaktionsgemisch wird mit 16 ml Diethylether verdünnt, der erhaltenen Niederschlag abfiltriert und zweimal mit je 15 ml kaltem Diethylether digeriert. Dieses Rohprodukt wird aus Chloroform/Aktivkohle umkristallisiert.
Ausbeute: 789 mg gelbe Kristalle (75% d.Th.)
DC: LM...PE:EtOH = 2:1; 0.3
Fp.: 226-227 ° C (Zers., Chloroform)
$^{1}$H-NMR: (DMSO-$d_6$)
d(ppm): 8.10- 7.61(m, 9H, TT-H7, Bz-H2,6, Py-H3,4,5, BF-H3,4,7); 7.59-7.43 (m, 3H, Bz-H3,4,5); 7.41 (ddd, 1H, BF-H6); 7.31 (ddd, 1H, BF-H5)
$^{13}$C-NMR: ($CF_3COOD$)
d(ppm): 173.4; 162.6; 159.8; 159.4; 151.1; 151.1; 147.4; 143.6; 142.0; 134.5; 134.3; 133.0; 133.0; 131.3; 129.2 128.7; 126.9; 122.2; 122.2; 120.6; 117.5; 116.2; 115.6 112.6; 43.3

**N-[6-(2-Formyl-phenoxymethyl)-2-pyridyl]-acetamid**

20 g (73,8 mMol) N-Acetyl-N-(6-brommethyl-2-pyridyl)-acetamid, 9 g (73,8 mMol) 2-Hydroxybenzaldehyd, 45,9 g (331,3 mMol) trockenes Kaliumcarbonat und 0,12 g (7,4 mMol) Kaliumjodid werden in 200 ml Dimethylformamid suspendiert und 5 Stunden auf 85 ° C erhitzt. Das Reaktionsgemisch wird abgekühlt, filtriert und das Lösungsmittel abgezogen.
Der Rückstand wird in 80 ml Dichlormethan aufgenommen, zwei mal mit je 40 ml 2n wässriger Natronlauge und zwei mal mit je 40 ml Wasser gewaschen, die organische Phase mit Natriumsulfat getrocknet, filtriert und das Lösungsmittel abgezogen.
Das Rohprodukt wird säulenchromatographisch (300g KG 60; $CH_2Cl_2$:EE = 2:1) gereinigt.
Ausbeute: 12 g hellgelbe Kristalle (60% d.Th.)
DC: LM...$CH_2Cl_2$:EE = 2:1; 0.5
Fp: 169-171 ° C (EtOH)
$^{1}$H-NMR: ($CDCl_3$)
d(ppm): 10,58 (s, 1H, Ph-C<u>H</u>O); 8,17(d, 1H, Py-H3); 8,03(s, 1H, Py-N<u>H</u>-); 7,86(d, 1H, Ph-H3); 7,75(dd, 1H, Py-H4); 7,53(dd, 1H, Ph-H5); 7,23(d, 1H, Py-H5); 7,05 (dd, 1H, Ph-H4); 6,98(d, 1H, Ph-H6); 5,17(s; 2H, Py-C<u>H</u>$_2$-O); 2,21 (s, 3H, C<u>H</u>$_3$-CO-)
$^{13}$C-NMR: (DMSO-$d_6$)
d(ppm): 188.6; 169.0; 159.8; 151.0; 138.1; 135.3; 127.7; 124.2; 120.4; 115.9; 112.5; 112.3; 70.0; 23.6

**N-[6-(2-Benzo[b]furyl)-2-pyridyl]-acetamid**

11 g (40,7 mMol) N-[6-(2-Formyl-phenoxymethyl)-2-pyridyl]-acetamid werden unter Stick-stoffatmosphäre 20 Minuten in einem Sandbad auf 300 ° C erhitzt.
Das erkaltete Rohprodukt wird chromatographisch (100g KG 60; $CH_2Cl_2$:EE = 2:1) gereinigt. Das erhaltene Produkt wird ohne weitere Reinigung in die nächste Stufe eingesetzt.
Ausbeute: 3,4 g farblose Kristalle (33 % d.Th.)
DC: LM...$CH_2Cl_2$:$Et_2O$ = 2:1; 0.6
Fp: 191-193 ° C (Aceton)
$^{1}$H-NMR: ($CDCl_3$)
d(ppm): 8,26(s, 1H, Py-N<u>H</u>-); 8,18(d, 1H, Py-H3); 7,79(dd, 1H, Py-H4); 7,62(d, 2H, BzFu-H4, Py-H5); 7,56(d, 1H, BzFu-H7); 7,39-7,20(m, 2H, BzFu-H5,H6); 7,32(s, 1H, BzFu-H3); 2,21 (s, 3H, C<u>H</u>$_3$-CO-)
$^{13}$C-NMR: ($CDCl_3$)
d(ppm): 168,8; 155,3; 154,5; 151,3; 147,4; 139,2; 128,6; 125,3; 123,3; 121,6; 115,9; 113,3; 111,6; 104,9; 24,7.

**6-(2-Benzo[b]furyl)-2-pyridinamin**

5,5 g (23,8 mMol) des zuvor erhaltenen Rohproduktes werden in 440 ml (190,4 mMol) 4 %iger wäßriger Schwefelsäure suspendiert und 90 Minuten zum Sieden erhitzt.
Das Gemisch wird abgekühlt, mit gesättigter, wäßriger Natriumhydrogen-carbonatlösung neutralisiert und drei mal mit je 200 ml Essigsäureethylester extrahiert. Die Vereinten organische Phasen werden mit 100 ml Wasser gewaschen, mit Natriumsulfat getrocknet, filtriert und das Lösungsmittel abgezogen.
Das Rohprodukt wird aus 60 ml Ethanol/Aktivkohle umkristallisiert.

Ausbeute 4,3 g farblose Kristalle (86 % d.Th.)
DC: LM...$CH_2Cl_2$:$Et_2O$ = 2:1; 0.5
Fp: 151-154 °C (EtOH)
$^1$H-NMR: (DMSO-$d_6$)
d(ppm): 7,70(d, 1H, BzFu-H4); 7,64( d, 1H, BzFu-H7); 7,51 (dd, 1H, Py-H4,); 7,34(dd, 1H, BzFu-H5); 7,34(s, 1H, BzFu-H3); 7,27(dd, 1H, BzFu-H6); 7,11(d, 1H, Py-H5); 6,49 (d, 1H, Py-H3); 6,20(s, 2H, Py-N<u>H</u>$_2$)
$^{13}$C-NMR: (DMSO-$d_6$)
d(ppm): 159,7; 155,8; 154,3; 146,3; 137,7; 128,5; 124,9; 123,1; 121,5; 111,2; 108,6; 108,0; 103,5.

**Beispiel 18**

**<u>N-[6-(2-Benzo[b]furyl)-2-pyridinyl]-4-hydroxy-2-methyl-6-(2-thienyl)-2H-thieno[2,3-e]-1,2-thiazin-3-carboxamid, 1,1-dioxid</u>**

700 mg (1.96 mMol) 4-Hydroxy-2-methyl-6-(2-thienyl)-2H-thieno[2,3-e]-1,2-thiazin-3-carbonsäuremethylester-1,1-dioxid und 412 mg (1.96 mMol) 6-(2-Benzo[b]furyl)-2-pyridinamin werden in 7 ml absolutem Xylol 10 Stunden zum Sieden erhitzt.
Das erkaltete Reaktionsgemisch wird mit 20 ml Diethylether verdünnt, der erhaltene Niederschlag abfiltriert und zweimal mit je 15 ml kaltem Diethylether digeriert. Das Rohprodukt wird aus Dimethylsulfoxid/Aktivkohle umkristallisiert.
Ausbeute: 821 mg gelbe Kristalle (78% d.Th.)
DC: LM.. .PE:EtOH = 2:1; 0.3
Fp.: 253-256 °C (Zers., DMSO)
$^1$H-NMR: (DMSO-$d_6$)
d(ppm): 8.08-7.90 (m, 2H, Py-H4,5); 7.90-7.58 (m, 7H, Th-H3,5, TT-H7, BF-H3,4,7, Py-H3); 7.41 (ddd, 1H, BF-H6); 7.31 (ddd, 1H, BF-H5); 7.20 (dd, 1H, Th-H4); 3.00 (s, 3H, C<u>H</u>$_3$)
$^{13}$C-NMR: ($CF_3COOD$)
d(ppm): 173.2; 162.9; 159.6; 152.2; 150.9; 147.4; 143.7; 141.7; 136.9; 133.1; 132.7; 132.2; 132.2; 131.2; 130.7; 128.6; 126.7; 121.7; 121.7; 120.4; 117.6; 116.0; 115.5; 112.4; 43.2

Beispiel 19

**<u>N-[6-(2-Benzo[b]furyl)-2-pyridinyl]-6-(2-furyl)-4-hydroxy-2-methyl-2H-thieno[2,3-e]-1,2-thiazin-3-carboxamid, 1,1-dioxid</u>**

700 mg (2.05 mMol) 6-(2-Furyl)-4-hydroxy-2-methyl-2H-thieno[2,3-e]-1,2-thiazin-3-carbonsäuremethylester-1,1-dioxid und 431 mg (2.05 mMol) 6-(2-Benzo[b]furyl)-2-pyridin-amin werden in 7 ml absolutem Xylol 9 Stunden zum Sieden erhitzt.
Das erkaltete Reaktionsgemisch wird mit 20 ml Diethylether verdünnt, der erhaltene Niederschlag abfiltriert und zweimal mit je 15 ml kaltem Diethylether digeriert. Das Rohprodukt wird aus Dioxan/Aktivkohle umkristallisiert.
Ausbeute: 687 mg gelbe Kristalle (64% d.Th.)
DC: LM...PE:EtOH = 2:1; 0.3
Fp.: 242-244 °C (Zers., Dioxan)
$^1$H-NMR: (DMSO-$d_6$)
d(ppm): 8.07-7.60 (m, 8H, Fu-H5, TT-H7, BF-H3,4,7, Py-H3,4,5); 7.41 (ddd, 1H, BF-H6); 7.31 (ddd, 1H, BF-H5); 7.25 (dd, 1H, Fu-H3); 6.72 (dd, 1H, Fu-H4); 3.00 (s, 3H, C<u>H</u>$_3$)
$^{13}$C-NMR: ($CF_3COOD$)
d(ppm): 172.8; 162.9; 159.3; 150.8; 150.6; 149.7; 147.9; 147.5; 146.6; 143.8; 141.5; 132.9; 132.2; 131.0; 128.3; 126.4; 120.6; 120.2; 117.6; 116.0; 115.4; 115.3; 113.5; 112.4; 42.9

**Beispiel 20**

**<u>6-(2-Furyl)-4-hydroxy-2-methyl-N-[6-phenyl-2-pyridinyl]-2H-thieno[2,3-e]-1,2-thiazin-3-carboxamid, 1,1-dioxid</u>**

500 mg (1.46 mMol) 6-(2-Furyl)-4-hydroxy-2-methyl-2H-thieno[2,3-e]-1,2-thiazin-3-carbonsäuremethylester-1,1-dioxid und 431 mg (1.46 mMol) 6-Phenyl-2-pyridinamin werden in 6 ml absolutem Xylol 4 Stunden

zum Sieden erhitzt.
Nach Abkühlen des Reaktionsgemisches wird der Niederschlag abfiltriert und zweimal mit je 15 ml kaltem Diethylether digeriert. Das Rohprodukt wird aus Toluol/Aktivkohle umkristallisiert.
Ausbeute: 360 mg gelbe Kristalle (51% d.Th.)
DC: LM...PE:EtOH = 2:1; 0.3
Fp.: 210-213 °C (Toluol)
$^1$H-NMR: (DMSO-$d_6$)
d(ppm): 8.15-8.05 (m, 2H, Bz-H2,6); 8.04-7.82 (m, 4H , Thaz-H7, Fu-H5, Py-H3,4); 7.78 (d, 1H, Py-H5); 7.57-7.44 (m, 3H, Bz-H3,4,5); 7.22 (d, 1H, Fu-H3); 6.69 (dd, 1H, Fu-H4)
$^{13}$C-NMR: (DMSO-$d_6$)
d(ppm): 166.4; 156.5; 153.7; 150.2; 146.8; 144.6; 140.1; 139.3; 138.9; 136.9; 133.2; 129.6; 128.8; 126.7; 117.8; 116.6; 114.5; 112.9; 110.0; 109.8; 39.6

**Beispiel 21**

**N-{6-[(2-Benzo[b]furyl)-methoxy]-2-pyridinyl}-6-(2-furyl)-4-hydroxy-2-methyl-2H-thieno[2,3-e]-1,2-thiazin-3-carboxamid-1,1-dioxid**

0.127 g (0.53 mmol) 6-[(2-Benzo[b]furyl)-methoxy]-2-pyridinamin und 0.18 g (0.53 mmol) 6-(2-Furyl)-4-hydroxy-2-methyl-2H-thieno[2,3-e]-1,2-thiazin-3-carbonsäure-methylester-1,1-dioxid werden in 10 ml abs. Xylol 4 Stunden zum Sieden erhitzt. Das Lösungsmittel wird abgezogen und das Rohprodukt dreimal mit je 5 ml heißem Diethylether digeriert.
Ausbeute: 0.14 g gelbe Kristalle (48% d. Th.)
DC: LM.. .Bz:Dx:AcOH = 10:1:1; 0.8
Fp: 190-191 °C (Toluol)
$^1$H-NMR: (DMSO-$d_6$)
δ(ppm): 7.95 (m, 3H Fu-H5, TT-H7, BF-H6); 7.69-7.52 (m, 3H, BF-H4,5,7) 7.39-7.14 (m, 3H, Py-H3,4, Fu-H3); 7.08 (s, 1H, BF-H3); 6.76- 6.67 (m, 2H, Fu-H4, Py-H5); 5.55 (s, 2H, -OC$\underline{H}_2$); 2.99 (s, 3H, -C$\underline{H}_3$)
$^{13}$C-NMR: (DMSO-$d_6$)
δ(ppm): 166.0; 161.1; 154.9; 154.3; 152.9; 147.9; 146.5; 144.6; 141.1; 139.3; 138.7; 132.0; 127.5; 124.6; 122.8; 121.2; 117.7; 112.8; 111.0; 110.3; 109.8; 108.2, 106.7; 106.5; 59.6; 39.4
Die Aminokomponente kann wie folgt hergestellt werden:

**6-[(2-Benzo[b]furyl)-methoxy]-2-brompyridin**

Zu 6.83 g (46.1 mmol) 2-Benzo[b]furanmethanol in 50 ml abs. Methanol werden 9.13 ml (50.7 mmol) einer 30 %igen Lösung von Natriummethanolat in abs. Methanol getropft. Das Lösungsmittel wird abgezogen und der Rückstand zusammen mit 10.87 g (45.9 mmol) 2,6-Dibrompyridin in 250 ml abs N,N-Dimethylformamid 3 Stunden bei 70-80 °C gerührt. Das Lösungsmittel wird abgezogen und der Rückstand in Essigsäureethylester aufgenommen. Die organische Phase wird mit 0.5 N Salzsäure gewaschen, über Natriumsulfat getrocknet, filtriert, das Lösungsmittel abdestilliert und das Rohprodukt aus Diethylether über Aktivkohle umkristallisiert.
Ausbeute: 7.84 g farblose Kristalle (56 % d. Th.)
DC: LM...PE:Bz = 4:1; 0.35
Fp.: 91-92 °C (Diethylether)
$^1$H-NMR ($CDCl_3$):
δ(ppm): 7.62-7.51 (m, 2H, BF-H4,7); 7.43 (dd, 1H, Py-H4); 7.37-7.18 (m, 2H, BF-H5,6); 7.10 (d, 1H, Py-H3); 6.84 (s, 1H, BF-H3); 6.76 (d, 1H, Py-H5); 5.80 (s, 2H, -OC$\underline{H}_2$)
$^{13}$C-NMR ($CDCl_3$):
δ(ppm): 162.3; 155.1; 152.4; 140.6; 138.2; 127.9; 124.6; 122.8; 121.2; 120.7; 111.3; 109.6; 106.9; 60.7

**6-[(2-Benzo[b]furyl)-methoxy]-2-pyridinamin**

4.48g (14.73 mmol) 6-[(2-Benzo[b]furyl)-methoxy]-2-brompyridin in 70 ml abs. Tetrahydrofuran werden bei -80 °C mit 5.89 ml (14.73 mmol) einer 2.5 N Lösung von Buthyllithium in n-Hexan versetzt und 30 Minuten nachgerührt.
Dann werden 2.13 g (14.73 mmol) 1-Azido-1-phenylethen in 28 ml abs. Tetrahydrofuran zugesetzt. Nach Angleichen an Raumtemperatur werden 200 ml 10 %ige Salzsäure zugesetzt, 20 Minuten gerührt und mit

festem Natriumhydroxid pH = 12 eingestellt. Die wäßrige Phase wird erschöpfend mit Ethylacetat extrahiert. Die organische Phase wird dann mit 2 N Salzsäure extrahiert, die wäßrige Phase mit festem Natriumhydroxid auf pH = 12 gestellt und rückgeschüttelt. Die Vereinten organischen Phasen werden über Natriumsulfat getrocknet, filtriert und das Lösungsmittel abgezogen. Die Isolierung des Produktes erfolgt chromatographisch. (Laufmittel: Chloroform; 100g Kieselgel)
Ausbeute: 0.38 g farblose Kristalle (11,5% d. Th.)
DC: LM...$CHCl_3$; 0.1
Fp: 82-83 °C (Toluol)
$^1$H-NMR ($CDCl_3$):
δ(ppm): 7.60-7.43 (m, 2H, BF-H4,7); 7.36 (dd, 1H, Py-H4); 7.30-7.16 (m, 2H, BF-H5,6); 6.77 (s, 1H, BF-H3); 6.18 (d, 1H, Py-H3); 6.08 (d, 1H, Py-H5); 5.40 (s, 2H, -OC<u>H</u>$_2$)
$^{13}$C-NMR ($CDCl_3$):
δ(ppm): 162.3; 156.9; 155.0; 153.7; 140.4; 128.1; 124.3; 122.6; 121.0; 111.2; 105.9; 100.1; 99.3; 59.8

**Beispiel 22**

<u>**N-{6-[(2-Benzo[b]thienyl)-methoxy]-2-pyridyl}-6-(2-furyl)-4-hydroxy-2-methyl-2H-thieno[2,3-e]-1,2-thiazin-3-carboxamid-1,1-dioxid**</u>

0.41 g (1.61 mmol) 6-[(2-Benzo[b]thienyl)-methoxy]-2-pyridinamin und 0.5 g (1.46 mmol) 6-(2-Furyl)-4-hydroxy-2-methyl-2H-thieno[2,3-e]-1,2-thiazin-3-carbonsäure-methylester-1,1-dioxid werden in 25 ml abs. Xylol 5 Stunden zum Sieden erhitzt. Das nach Abkühlen ausfallende Produkt wird abfiltriert, aus Toluol/Aktivkohle umkristallisiert und dreimal mit wenig eiskaltem Diethylether digeriert.
Ausbeute: 0.58 g gelbe Kristalle (70 % d. Th.)
DC: LM...Bz:Dx:AcOH = 10:1:1; 0.8
Fp: 194-195 °C (Toluol)
$^1$H-NMR (DMSO-$d_6$):
δ(ppm): 7.99-7.7 (m, 5H, FuH5, TT-H7, BT-H5,6,7); 7.69-7.52 (m, 2H, BT-H4, BT-H3); 7.42-731 (m, 2H, Py-H3,4); 7.27 (d, 1H, Fu-H3) 6.75-6.68 (m, 2H, Fu-H3, Py-H5); 5.75 (s, 2H, OC<u>H</u>$_2$); 3.05 (s, 3H, -C<u>H</u>$_3$)
$^{13}$C-NMR (DMSO-$d_6$):
δ(ppm):166.0; 161.2; 155.0; 148.1; 146.7; 144.7; 141.3; 140.2; 139.6; 139.4; 138.9; 132.2; 124.6; 124.4; 124.2; 123.7; 122.4; 117.9; 113.0; 110.5; 108.2; 109.9 106.6; 64.9; 62.5; 39.6
Die Aminokomponente kann wie folgt hergestellt werden:

<u>**6-[(2-Benzo[b]thienyl)-methoxy]-2-brompyridin**</u>

7.01g (42.7 mmol) 2-Benzo[b]thiophenmethanol werden in 50 ml abs. Methanol mit 8.75ml (42.7 mmol) einer 30 %igen Lösung von Natriummethanolat in abs. Methanol versetzt und das Lösungsmittel bei Raumtemperatur abgezogen. Der Rückstand wird zusammen mit 10.11 g (42.2 mmol) 2,6-Dibrompyridin in 250 ml abs Dimethylformamid 3 Stunden bei 70-80 °C gerührt. Nach Abziehen des Lösungsmittels wird der Rückstand in Essigsäureethylester aufgenommen, mit 0.5N Salzsäure gewaschen, über Natriumsulfat getrocknet, filtriert und das Lösungsmittel abgezogen Das Rohprodukt wird aus Diethylether/Aktivkohle umkristallisiert.
Ausbeute: 10.37 g farblose Kristalle (75 % d. Th.)
DC: LM...Pe:Bz = 4:1; 0.35
Fp.: 82-83 °C (Diethylether)
$^1$H-NMR ($CDCl_3$):
δ(ppm): 7.88-7.71 (m, 2H, BT-H4,7); 7.40 (dd, 1H, Py-H4); 7.39-7.28 (m, 3H, BT-H3,5,6); 7.11 (d, 1H, Py-H3); 6.76 (d, 1H, Py-H5); 5.63 (s, 2H, - OC<u>H</u>$_2$)
$^{13}$C-NMR ($CDCl_3$):
δ(ppm): 162.4; 140.6; 140.4; 139.5; 139.2; 138.3; 124.5; 124.2; 124.2; 123.7; 122.3; 120.8; 109.7; 63.5

<u>**6-[(2-Benzo[b]thienyl)-methoxy]-2-pyridamin**</u>

6.37g (19.89 mmol) 6-[(2-Benzo[b]thienyl)-methoxy]-2-brompyridin in 70 ml abs. Tetrahydrofuran werden bei -80 °C mit 7.96 ml (19.89 mmol) einer 2.5 N Lösung von Butyllithium in n-Hexan versetzt und 30 Minuten nachgerührt. Dann werden bei -80 °C 2.88 g (19.89 mmol) 1 -Azido-1-phenylethen in 28 ml abs. Tetrahydrofuran zugesetzt. Nach Angleichen an Raumtemperatur werden 300 ml 10 %ige Salzsäure

zugesetzt, 20 Minuten gerührt und mit Natriumhydroxidplätzchen auf pH = 12 gestellt. Die wäßrige Phase wird 9 mal mit je 30 ml Ethylacetat extrahiert. Die Vereinten organischen Phasen werden über Natriumsulfat getrocknet, filtriert und das Lösungsmittel abgezogen. Die Isolierung des Produktes erfolgt säulen-chromatographisch. (Laufmittel: Chloroform; 210 g Kieselgel).
Ausbeute: 1.49 g farblose Kristalle (30 % d. Th.)
DC: LM...$CHCl_3$; 0.1
Fp: 91-94 °C
$^1$H-NMR($CDCl_3$):
δ(ppm): 7.86-7.69 (m, 2H, BT-H4,7); 7.39 (dd, 1H, Py-H4); 7.33-7.28 (m, 3H, BT-H3,5,6); 6.28 (d, 1H, Py-H3); 6.10 (d, 1H, Py-H5); 5.56 (s, 2H, - $OC\underline{H}_2$)
$^{13}$C-NMR (CDCl3):
δ(ppm): 162.2; 156.9; 140.9 140.4; 140.3; 139.2; 124.1; 124.1; 123.4; 123.2; 122.3; 100.1; 99.1; 62.4

**Beispiel 23**

**6-(2-Benzo[b]furanyl)-4-hydroxy-2-methyl-N-(6-phenyl-2-pyridinyl)-2H-thieno[2,3-e]-1,2-thiazin-3-carboxamid, 1,1-dioxid**

0.20 g (0.51 mMol) 6-(2-Benzo[b]furanyl)-4-hydroxy-2-methyl-2H-thieno[2,3-e]-1,2-thiazin-3-carbonsäuremethylester-1,1-dioxid und 0.09 g (0.51 mMol) 6-Phenyl-2-pyridinamin werden in 2 ml absolutem Xylol 7 Stunden zum Sieden erhitzt.
Das erkaltete Reaktionsgemisch wird filtriert und mit 5 ml kaltem Diethylether digeriert. Das Rohprodukt wird aus Dimethylsulfoxid/Aktivkohle umkristallisiert.
Ausbeute: 70 mg gelbe Kristalle (25,8% d.Th.)
DC: LM...PE:EtOH = 3:1; 0.3
Fp.: Zers. ab 225 °C (DMSO)
$^1$H-NMR: (DMSO-$d_6$)
d(ppm): 8.16-8,08 (m, 3H, Bzfu-H3, Py-H3,5); 8,06-7,89 (m, 2H, Bzfu-H4,7); 7,84-7,63 (m, 4H, Bz-H3,4,5, Py-H4); 7,58-7,47 (3H, Bz-H2,6, Thaz-H7); 7,47-7,28 (m, 2H, Bzfu-H5,6); 3,02 (s, 3H, OCH3)
Das Ausgangsmaterial kann wie folgt hergestellt werden:

**4-Methylbenzolsulfonsäure, [2-cyano-1-(2-benzo[b]furyl)-ethenyl]-ester**

Zu 24,00 g (129,60 mMol) b-Oxo-2-benzo[b]furanpropannitril und 14,42 g (142,56 mMol) N-Methylmorpholin in 180 ml absolutem Dichlormethan werden bei Raumtemperatur 27.18 g (142,56 mMol) p-Toluolsulfonsäurechlorid in 50 ml absolutem Dichlormethan getropft und eine Stunde nachgerührt.
Das Reaktionsgemisch wird mit 2x100 ml Wasser und einmal mit 50 ml 2 n Salzsäure gewaschen. Die organische Phase wird über $Na_2SO_4$/Aktivkohle getrocknet, filtriert und das Lösungsmittel abdestilliert. Den erhaltenen Feststoff wird mit kaltem Diisopropylether digeriert.
Ausbeute: 35,92 g farblose Kristalle (81,7% d.Th.)
DC: *LM*...PE:EE = 5:1; 0.4
Fp.: 143-145 °C (DIPE)
$^1$H-NMR: (CDCl3)
d(ppm): 8,03 (d, 2H Bz-H2,6); 7.65 (d, 1H, Bzfu-H4) 7,55-7,24 (m, 6H, Bz-H3,5, Bzfu-H3,5,6,7); 5,91 (s, 1H, = CH); 2.50 (s, 3H, $C\underline{H}_3$)
$^{13}$C-NMR: (DMSO-$d_6$)
d(ppm): 155,1; 150,3; 147,0; 146,8; 130,9; 130,4; 128,5; 127,9; 127,1; 124,2; 122,8; 114,1; 111,9; 111,5; 90,6; 21,1

**3-Amino-5-(2-benzo[b]furanyl)-2-thiophencarbonsäuremethylester**

19,67 g (109,53 mMol) Natriummethanolat 30%ig in 350 ml absolutem Methanol werden bei 15-20 °C mit 11,63 g (109,53 mMol) Thioglycolsäuremethylester versetzt und 20 Minuten bei Raumtemperatur gerührt. Anschließend gibt man in einer Portion 35,4 g (104,31 mMol) 4-Methylbenzolsulfonsäure-[2-cyano-1-(2-benzo[b]furanyl)-ethenyl]-ester zu und rührt weitere 1½ Stunden.
Nach dem Abziehen des Lösungsmittels wird zwischen 300 ml Wasser und 200 ml Dichlormethan verteilt, noch dreimal mit 100 ml Dichlormethan extrahiert und die organische Phase über $Na_2SO_4$/Aktivkohle getrocknet, filtriert und das Lösungsmittel abgezogen. Das Rohprodukt wird aus Ethanol/Aktivkohle umkristallisiert.
Ausbeute: 17,10 g farblose Kristalle (60,0% d.Th.)
DC (Amin): *LM*...PE:EE = 2:1; 0.60
Fp.: 160-162 ° C (Ethanol)
$^1$H-NMR: (DMSO-$d_6$)
d(ppm): 7,70-7,59 (m, 2H, Bzfu-H4,7); 7,42-7,24 (m, 3H, Bzfu-H3,5,6); 7,10 (s, 1H, Th-H4); 3,37 (s, 3H, OCH3)
$^{13}$C-NMR: (DMSO-$d_6$)
d(ppm): 163,9; 155,0; 154,2; 147,7; 135,9; 128,3; 125,5; 123,6; 121,5; 116,9; 111,1; 104,0; 97,4; 51,1

**5-(Benzo[b]furanyl)-3-chlorsulfonyl-2-thiophencarbonsäuremethylester** 15,76 g (57,69 mMol) 3-Amino-5-(2-benzo[b]furanyl)-2-thiophen-carbonsäuremethylester 50 ml abs. Diethylether werden mit 41,20 g 11,5 %ige etherische Salzsäure 10 Minuten gerührt und das Lösungsmittel abgezogen.
Zu einer Suspension von 16,80 g (54,23 mMol) dieses Hydrochlorides in 100 ml conz. Salzsäure werden während 1 Stunde bei 0 ° C 4,18 g (60,53 mMol) Natriumnitrit in 8 ml Wasser unter die Flüssigkeitsoberfläche eingebracht und zwei Stunde nachgerührt.
Diese Lösung wird zu einem Gemisch aus 450 ml gesättigter Schwefeldioxidlösung in Eisessig (~ 40%ig) und 29 ml einer gesättigten wäßrigen Kupfer(II)chloridlösung gegossen, auf 30 ° C erwärmt und 1 Stunden nachgerührt.
Das Reaktionsgemisch wird auf 600 l Eiswasser gegossen, mit 4x100 ml Dichlormethan extrahiert und die Vereinten organischen Phasen mit 2x100 ml Wasser gewaschen. Die organische Phase wird über $Na_2SO_4$/Aktivkohle getrocknet, filtriert, das Lösungsmittel abgezogen und das Rohprodukt wird mit kaltem Diethylether digeriert..
Ausbeute: 12,60 g gelbe Kristalle (65,5% d.Th.)
DC: *LM*...PE:EtOH = 2:1; 0.8
Fp.: 158-160 ° C (Ether)
$^1$H-NMR: ($CDCl_3$)
d(ppm): 7,90 (s, 1H, Th-H4); 7,63 (d, 1H, Bzfu-H4); 7,53 (d, 1H, Bzfu-H7); 7,44-7,25 (m, 2H, Bzfu-H5,6); 7,12 (s, 1H, bzfu-H3)
$^{13}$C-NMR: ($CDCl_3$)
d(ppm): 161,2; 154,8; 153,0; 149,7; 134,4; 129,0; 128,3; 128,25; 126,1; 124,3; 122,2; 111,7; 104,7; 53,1

**5-(2-Benzo[b]furanyl)-3-[N-(methoxycarbonylmethyl)-sulfamoyl]-2-thiophencarbonsäure-methylester**

Eine Suspension von 11,6 g (32,51 mMol) 5-(2-Benzo[b]furanyl)-3-chlorsulfonyl-2-thiophencarbonsäure-methylester 5,94 g (43 mMol) Kaliumcarbonat und 5,4 g (43 mMol) Glycinmethylesterhydrochlorid in 60ml abs. Dichlormethan und 18 ml absolutem Methanol wird 6 Stunde zum Sieden erhitzt.
Das Reaktionsgemisch wird auf 300 ml eiskalte 2n Salzsäure gegossen, das entstandene Kristallisat abfiltriert und dreimal mit je 50 ml eiskaltem Wasser digeriert. Das Rohprodukt wird aus Methanol/Aktivkohle umkristallisiert.
Ausbeute: 10,97g gelbe Kristalle (76% d.Th.)
DC: *LM*...PE:EE = 2:1; 0.45
Fp.: 156-158 ° C (Methanol)
$^1$H-NMR: ($CDCl_3$)
d(ppm): 7,96-7,83 (m, 2H, NH, Th-H4); 7,75-7,60 (m, 3H, Bzfu-3,4,7); 7,46-7,25 (m, 2H, Bzfu-H5,6); 3,97 (d, 2H, CH2); 3,88 (s, 3H, Th-COOCH3); 3,55 (s, 3H, CH2COOCH3)

**6-(2-Benzo[b]furanyl)-4-hydroxy-2H-thieno[2,3-e]-1,2-thiazin-3-carbonsäure-methylester-1,1-dioxid**

Zu 2,17 g (19,34 mMol) Kalium-tert.-butanolat in 26 ml absolutem Tetrahydrofuran tropft man bei -10 bis -5 ° C 3,6 g (8,79 mMol) 5-(2-Benzo[b]furanyl)-3-[N-(methoxycarbonylmethyl)-sulfamoyl]-2-thiophencarbonsäuremethylester in 36 ml absolutem Tetrahydrofuran und rührt eine 1 Stunde nach.
Nach Zusatz von 159 ml eiskalter 2n Salzsäure wird das Rohprodukt mit 6x100 ml extrahiert, das Lösungsmittel abdestilliert 2x mit 100 ml Benzol eingedampft.
Dieses Rohprodukt wird mit wenig kaltem Acetonitril digeriert.
Ausbeute: 1,60 g gelbe Kristalle (48,2% d.Th.)
DC: *LM*...Bz:MeOH = 3:1; 0.6

Fp.: Zers. ab 235 ° C (Methanol)
$^{1}$H-NMR: (DMSO-$d_6$)
d(ppm): 8,12 (s, 1H, Thaz-H7); 7,75-7,60 (m, 3H, Bzfu-H3,4,7); 7,45-7,24 (m, 2H, Bzfu-H5,6); 3,90 (s, 3H, OCH3)

**6-(2-Benzo[b]furanyl)-4-hydroxy-2-methyl-2H-thieno[2,3-e]-1,2-thiazin-3-carbonsäure-methylester-1,1-dioxid**

Zu einer Suspension von 49 mg (2,04 mMol) Natriumhydrid in 1,2 ml absolutem DMF werden bei 7 ° C 700 mg (1,85 mMol) 6-(2-Benzo[b]furanyl)-4-hydroxy-2H-thieno[2,3-e]-1,2-thiazin-3-carbonsäuremethylester-1,1-dioxid in 2,4 ml absolutem DMF getropft und eine Stunde nachgerührt. Die erhaltene Lösung wird mit 0.32 g (2,23 mMol) Jodmethan versetzt und 20 Stunden bei Raumtemperatur gerührt
Nach Zugabe von 20 ml eiskalter 2n Salzsäure wird das Rohprodukt mit 4x20 ml Dichlormethan extrahiert, die vereinten organischen Phasen getrocknet, filtriert und das Lösungsmittel abgezogen. Dieses wird mit 2x20 ml heißem Ethanol digeriert.
Ausbeute: 0,35 g gelbe Kristalle (48,2% d.Th.)
DC: *LM*...Bz:MeOH = 3:1; 0.8
Fp.: Zers. ab 220 ° C (roh)
$^{1}$H-NMR: (DMSO-$d_6$)
d(ppm): 8,22 (s, 1H, Thaz-H7); 7,79-7,62 (m, 3H, bzfu-H3,4,7); 7,48-7,27 (m, 2H, Bzfu-H5,6); 3,90 (s, 3H, OCH3); 3,02 (s, 3H, NCH3)

**Beispiel 14**

Die Bildung von Prostaglandin $D_2$ durch Neutrophile wurde als Maß für die Cyclooxygenase-Aktivität verwendet und die Bildung von Leucotrien $B_4$ als Maß für die 5-Lipoxygenase-Aktivität.
Männliche Sprague Dawley Ratten (250-300 g) wurde lamda-Carrageenan 1mg intraperitoneal (gelöst in 0,5 ml dest. Wasser) injiziert.
Nach 16 Stunden wurden die Ratten durch Exposition in Diethylether getötet.
15 ml Hanks balanced salt solution (HBSS) wurden i.p. injiziert, die Neutrophilen durch Absaugen geerntet (10 ml), zentrifugiert (5 min., 100 g, 4 ° C), die überstehende Lösung dekantiert und die Zellen in HBSS bei 4 ° C bis zu einer Konzentration von $5x10^6$ Zellen/ml resuspendiert.
400 µl Zellsuspension ($2x10^6$ Zellen), 0,5 µg Verbindung gelöst in DMSO und 49,5 µl HBSS wurden 5 min, bei 37 ° C inkubiert` Dann wurden 50 µl A23187 (2 µmol/l Endkonzentration) zugefügt und anschließend wiederum 5 min. bei 37 ° C inkubiert.
Die Reaktion wurde durch Zentrifugieren 10000 g, 3 s gestoppt und die überstehende Flüssigkeit in vorgekühlte Plastikröhrchen überführt und vor Beginn der Radioimmunoassay-Messung im Eisbad max. 1 h belassen.
$PGD_2$ und $LTB_4$ wurde nach Verdünnung mit HBSS mit Hilfe kommerzieller RIA-Kits gemessen.
Als Vergleichssubstanz diente 6-Chlor-2-methyl-N-(2-pyridyl)-2H-thieno[2,3-e]-1,2-thiazin-3-carbonsäureamid-1,1-dioxid ("*LORNOXICAM*", Vbg. A).

| | IC50 (µmol/l) | |
|---|---|---|
| Vbg. | $PGD_2$ | $LTB_4$ |
| A | 0,02 | >10 |
| 1 | 0,017 | >10 |
| 2 | 0,027 | >10 |
| 3 | 0,017 | 5,2 |
| 4 | 3,4 | 0,47 |
| 5 | 0,27 | 0,66 |
| 6 | 3,6 | 0,58 |
| 7 | 1,6 | 0,73 |
| 8 | 0,1 | 2,2 |
| 9 | 0,039 | 1,6 |
| 11 | 0,2 | 2,1 |
| 12 | 0,19 | 2,7 |
| 14 | 0,038 | >10 |
| 15 | 0,0035 | >10 |
| 16 | 0,0096 | >10 |
| 17 | 0,19 | 1,3 |
| 18 | 0,068 | 1,3 |
| 19 | 0,039 | 1,4 |

## Patentansprüche

1. Neue Thienothiazin-Derivate der allgemeinen Formel

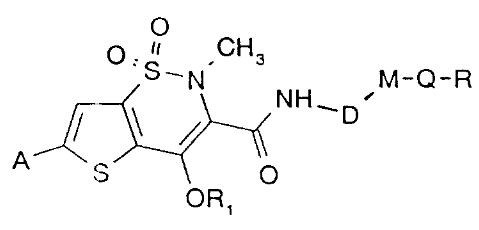

(I),

worin:

**A** Niederalkyl, perfluoriertes Niederalkyl, Niederalkyloxy, Halogen, Nitro, Cyano oder einen mono- oder polycyclischen 5-12 gliedrigen, gegebenenfalls teilweise hydrierten Aryl- oder Heteroarylrest mit 1-4 Heteroatomen wie O, S und N, der gegebenenfalls mit Niederalkyl, Perfluorniederalkyl, Aryl, Heteroaryl, substituiertem Aryl, substituiertem Heteroaryl, Halogen, Niederalkoxy, Aryloxy, substituiertem Aryloxy, Heteroaryloxy, substituiertem Heteroaryloxy und Nitro substituiert sein kann, in denen die angesprochenen Substituenten in den substituiertem Aryl, substituiertem Heteroaryl, substituiertem Aryloxy und substituiertem Heteroaryloxy Halogen, Niederalkyl, Perfluorniederalkyl, Niederalkoxy u.ä. bedeuten;

**D** einen 2-Pyridylrest oder einen Rest

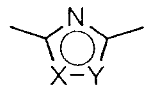 ,

wobei **X** und **Y** unabhängig voneinander CH, $NR^6$, O oder S, mit $R^6$ Wasserstoff oder Niederalkyl bedeuten,
**M** eine Einfachbindung, eine geradkettige oder verzweigte Kohlenstoffkette mit 1-12 Kohlenstoffatomen in der Kette, wobei diese Kette ein oder mehrere Doppel- und/oder Dreifachbindungen und/oder ein oder mehrere Heteroatome, wie O, S und N, enthalten kann, einen 5-12 gliedrigen mono- oder polycyclischen gegebenenfalls teilweise hydrierten Aryl- oder Heteroarylrest mit 1-4 Heteroatomen wie O, S und N, der durch Halogen, Niederalkyl oder Niederalkoxy substituiert sein kann;
**Q** eine Einfachbindung oder ein Heteroatom wie O, S und N;
**R** Wasserstoff, einen 5-12 gliedrigen mono- oder polycyclischen Aryl-oder Heteroarylrest mit 1-4 Heteroatomen wie O, S und N, der gegebenenfalls teilweise hydriert sein kann, oder auch ein oder mehrfach mit Halogen, Niederalkyl oder Niederalkoxy substituiert sein kann;
und $\mathbf{R_1}$ Wasserstoff, oder

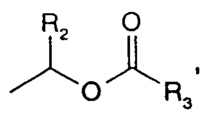 ,

worin $\mathbf{R_2}$ Niederalkyl und $\mathbf{R_3}$ Niederalkyl, Aryl oder $-OR_4$ mit $\mathbf{R_4}$ Niederalkyl, Cycloalkyl mit 4-8 Kohlenstoffatomen oder Aryl; bedeuten, sowie ihre pharmazeutisch verwendbaren Salze.

2. Verbindungen nach Anspruch 1, in denen **D** die Bedeutung 2-Pyridyl hat und die Substituenten **-M-Q-R** an der 6-Stellung des Pyridinkernes verknüpft sind.

3. Verbindungen nach Anspruch 1, bei denen **A** Chlor oder 2-Furanyl bedeutet.

4. Verbindungen nach Anspruch 3, bei denen **X** O oder S und **Y** CH bedeutet.

5. Ein Verfahren zur Herstellung von Verbindungen der allgemeinen Formel (I), welches dadurch gekennzeichnet ist, das man eine Verbindung der allgemeinen Formel

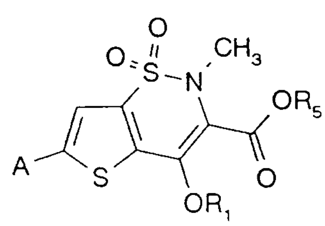 (II),

worin $\mathbf{R_1}$ Wasserstoff, $\mathbf{R_5}$ Niederalkyl bedeutet und **A** obige Bedeutung hat, mit einer Verbindung der allgemeinen Formel

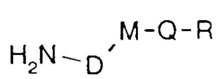 (III),

worin **D, M, Q** und **R** obige Bedeutung haben, umsetzt und die so erhaltenen Verbindungen der allgemeinen Formel (I) für $\mathbf{R_1}$ = Wasserstoff gegebenenfalls in ihre pharmazeutisch verwendbaren Salze überführt oder mit einer Verbindung der allgemeinen Formel

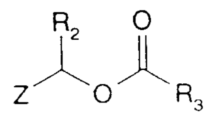 (IV),

worin **Z** Chlor oder Brom bedeutet und $\mathbf{R_2}$ und $\mathbf{R_3}$ obige Bedeutung haben, zu den Verbindungen der allgemeinen Formel (I) mit $\mathbf{R_1}$ nicht Wasserstoff umsetzt.

**6.** Pharmazeutische Präparate, enthaltend Verbindungen der allgemeinen Formel (I) nach Anspruch 1, sowie deren Salze in Kombination mit üblichen galenischen Hilfs- und/oder Trägerstoffen.

**7.** Pharmazeutische Präparate nach Anspruch 6, in Kombination mit anderen therapeutisch wertvollen Verbindungen sowie Hilfs- und/oder Trägerstoffen.

**8.** Verbindungen nach Anspruch 1, zur Verwendung als Wirkstoffe für Arzneimittel zur Behandlung von Entzündungen und Schmerzzuständen.

**9.** Verbindungen nach Anspruch 1, zur Verwendung als Antiinflammatorikum und Analgeticum.

Neue Thienothiazin-Derivate der allgemeinen Formel

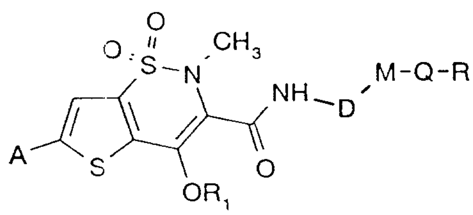 (I),

worin:

**A** Niederalkyl, perfluoriertes Niederalkyl, Niederalkyloxy, Halogen, Nitro, Cyano oder einen mono- oder polycyclischen 5-12 gliedrigen, gegebenenfalls teilweise hydrierten Aryl- oder Heteroarylrest mit 1-4 Heteroatomen wie O, S und N, der gegebenenfalls mit Niederalkyl, Perfluorniederalkyl, Aryl, Heteroaryl, substituiertem Aryl, substituiertem Heteroaryl, Halogen, Niederalkoxy, Aryloxy, substituiertem Aryloxy, Heteroaryloxy, substituiertem Heteroaryloxy und Nitro substituiert sein kann, in denen die angesprochenen Substituenten in den substituiertem Aryl, substituiertem Heteroaryl, substituiertem Aryloxy und substituiertem Heteroaryloxy Halogen, Niederalkyl, Perfluorniederalkyl, Niederalkoxy u.ä. bedeuten;

**D** einen 2-Pyridylrest oder einen Rest

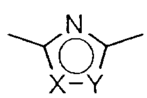 ,

, wobei **X** und **Y** unabhängig voneinander CH, $NR^6$, O oder S mit $R^6$ Wasserstoff oder Niederalkyl bedeuten,

**M** eine Einfachbindung, eine geradkettige oder verzweigte Kohlenstoffkette mit 1-12 Kohlenstoffatomen in der Kette, wobei diese Kette ein oder mehrere Doppel- und/oder Dreifachbindungen und/oder ein oder mehrere Heteroatome, wie O, S und N, enthalten kann, einen 5-12 gliedrigen mono- oder polycyclischen gegebenenfalls teilweise hydrierten Aryl- oder Heteroarylrest mit 1-4 Heteroatomen wie O, S und N, der durch Halogen, Niederalkyl oder Niederalkoxy substituiert sein kann;

**Q** eine Einfachbindung oder ein Heteroatom wie O, S und N;

**R** Wasserstoff, einen 5-12 gliedrigen mono- oder polycyclischen Aryl- oder Heteroarylrest mit 1-4 Heteroatomen wie O, S und N, der gegebenenfalls teilweise hydriert sein kann, oder auch ein oder mehrfach mit Halogen, Niederalkyl oder Niederalkoxy substituiert sein kann;

und $\mathbf{R_1}$ Wasserstoff, oder

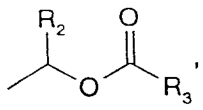

worin **$R_2$** Niederalkyl und **$R_3$** Niederalkyl, Aryl oder $-OR_4$ mit **$R_4$** Niederalkyl, Cycloalkyl mit 4-8 Kohlenstoffatomen oder Aryl; bedeuten, sowie ihre pharmazeutisch verwendbaren Salze.

Europäisches Patentamt

# EUROPÄISCHER RECHERCHENBERICHT

Nummer der Anmeldung
EP 94 11 9114

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP-A-0 103 142 (F. HOFFMANN-LA ROCHE & CO.)<br>*Insgesamt*<br>--- | 1-9 | C07D513/04<br>A61K31/54<br>//(C07D513/04, 333:00,279:00) |
| X | CH-A-619 236 (F.HOFFMANN-LA ROCHE & CO.)<br>*Insgesamt*<br>--- | 1-9 | |
| X | EP-A-0 313 935 (CL PHARMA AKTIENGESELLSCHAFT*)<br>*Insgesamt*<br>--- | 1-9 | |
| X | GB-A-1 519 811 (F. HOFFMANN-LA ROCHE & CO.)<br>*Insgesamt*<br>--- | 1-9 | |
| D,A | EP-A-0 001 113 (F. HOFFMANN-LA ROCHE & CO.)<br>*Insgesamt*<br>& US-A-4 180 662 (PFISTER, RUDOLF,DR. ET AL)<br>--- | 1-9 | RECHERCHIERTE SACHGEBIETE (Int.Cl.6) |
| P,X | EP-A-0 576 906 (CHEMISCHE PHARMAZEUTISCHE FORSCUNGS-GESELLSCHAFT M.B.H.)<br>*Insgesamt*<br>----- | 1-9 | C07D |

Der vorliegende Recherchenbericht wurde für alle Patentansprüche erstellt

| Recherchenort | Abschlußdatum der Recherche | Prüfer |
|---|---|---|
| DEN HAAG | 2. Februar 1995 | Luyten, H |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument